# EUROPEAN PATENT APPLICATION

(11) **EP 2 272 524 A2**
(43) Date of publication of application: **12.01.2011**
(21) Application number: 10075185.8
(22) Date of filing: 17.03.2005
(51) Int. Cl.: A61K 38/17, C07K 14/705, A61P 3/04, A61P 9/00, A61P 19/00

(54) **Y2 selective receptor agonists for therapeutic interventions**

(30) Priority: 17.03.2004 DK 200400436; 01.04.2004 US 558932 P; 06.07.2004 US 585964 P; 12.01.2005 GB 0500586
(62) Divisional of application: 05730285.3
(71) Applicant: 7TM Pharma A/S, 2970 Horsholm (DK)
(72) Inventor: Schwartz, Thue, 2970 Horsholm (DK)
(74) Representative: Walls, Alan James

(57) **Abstract**

Y receptor agonists selected from
[Lys4,Leu17,Gln34]PP,
[Cys2, D-Cys27]PYY2-36,
Lys4,Leu17,Leu30,Gln34]PP
[Lys4,Nle17,Nle30,Gln34]PP
[Lys4,Gln34]PP
[Cys2,D-Cys27]PYY
[Cys2,D-Cys27]NPY
[Cys2,Ile3,D-Cys27,Val31]NPY
[Cys2,Ile3,D-Cys27,Leu28,Val31]NPY
[Cys2,Ile3,Nle17,D-Cys27,Nle28,Val31]NPY
and N-acylated and conservatively substituted analogues thereof. are selective for the Y2 receptor over the Y1 and Y4 receptors, and their use in the treatment of conditions responsive to activation of Y2 receptors, are disclosed.

## Description

### FIELD OF THE INVENTION

The invention relates to peptide or peptidic compounds that act as selective agonists of the Y2 relative to the Y1 and Y4 receptors, and to their use in treatment of conditions responsive to activation of Y2 receptors, for example in treatment of obesity and overweight, and conditions in which these are considered contributory factors and for induction of angiogenesis.

### BACKGROUND TO THE INVENTION

**The PP-fold family of peptides** - NPY (Neuropeptide Y) (human sequence - SEQ ID. No:1), PYY (Peptide YY) (human sequence- SEQ ID. No:2), and PP (Pancreatic Polypeptide) (human sequence - SEQ ID. No:3), are naturally secreted homologous, 36 amino acid, C-terminally amidated peptides, which are characterized by a common three-dimensional, structure - the PP-fold - which is surprisingly stable even in dilute aqueous solution and is important for the receptor recognition of the peptides.

Initially the X-ray structure of avian PP was characterized in great detail through X-ray crystallographic analysis down to a resolution of 0.98 A and the unique structure obtained its name from this peptide *(*Blundell et al. 1981 Proc.Natl.Acad.Sci.USA 78: 4175-79*;* Glover et al. 1984, Eur.J.Biochem. 142: 379-85*).* Subsequently, the PP-fold structure of other members of the family have been analysed through especially NMR spectroscopic analysis. Both X-ray and NMR analysis are obviously performed in very concentrated or solid conditions; however, detailed circular dichroism analysis suggests that NPY and PP even in aqueous solution adopt the PP-fold structure, which is unusual for such a small peptide *(*Fuhlendorff et al. 1990 J. Biol. Chem. 265: 11706-12*).* Importantly, analysis of the proteolytic stability of the peptides and fragments and analogs of these strongly indicate that for example the full length PP1-36 even in dilute aqueous solution is held in a folded configuration which protects it from degradation by certain enzymes which readily and rapidly degrade analogs which cannot adopt the PP-fold structure due to minor substitutions *(*Schwartz et al. 1990 Annals NY Acad. Sci. 611: 35-47*).*

The PP-fold structure common to NPY, PYY and PP consists of 1) an N-terminal polyproline-like helix (corresponding to residues 1 through 8 with Pro2, Pro5, and

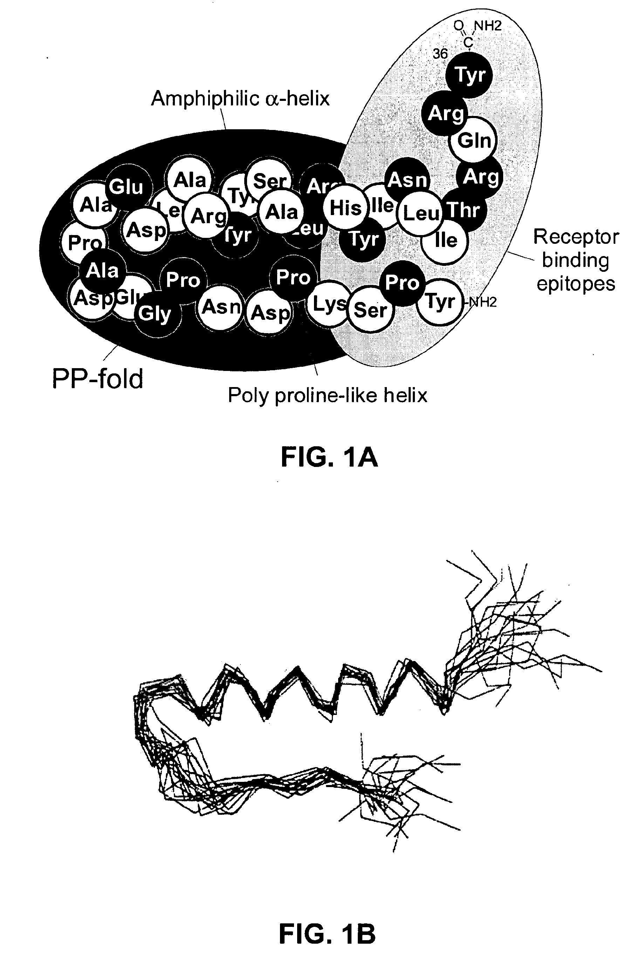

Pro8) followed by 2) a type I beta-turn region (corresponding to residues 9 through 12) followed by 3) an amphiphilic alpha-helix (residues 13-30) which lies anti-parallel to the polyproline helix with an angle of about 152 degrees between the helical axes, and 4) a C-terminal hexapeptide (residues 31-36). The folded structure is stabilized through hydrophobic interactions between side chains of the amphiphilic alpha-helix which are closely interdigitating with the three hydrophobic proline residues (*Schwartz et al 1990*). Besides key residues in the receptor recognizing C-terminal hexapeptide it is the core hydrophobic residues, which stabilize the PP-fold structure, which are conserved across the family of PP-fold peptides. Fig. 1A depicts the NPY sequence, with residues which are conserved amongst NPY, PYY and PP shown as white text on dark background. Fig. 1A also illustrates the elements of the PP-fold structure described above. The C-terminal hexapeptide, which is important for receptor recognition is generally believed to be unstructured, but the PP fold provides a stable scaffold, which presents the C-terminal hexapeptide to the receptors (illustrated in Fig. 1 B), which to variable degree are dependent or independent also upon parts of the N-terminus of the peptides. NMR spectroscopic analysis has demonstrated that the far C- and the N-terminal parts of for example NPY are rather mobile, meaning that the PP-fold is constantly in danger of being "unzipped" from the free terminal end.

**NPY** is a very wide-spread neuropeptide with multiple actions in various parts of both the central and peripheral nervous system acting through a number of different receptor subtypes in man: Y1, Y2, Y4 and Y5. The main NPY receptors are the Y1 receptor, which generally is the post-synaptic receptor conveying the "action" of the NPY neurones and the Y2 receptor which generally is a pre-synaptic, inhibitory receptor. This is also the case in the hypothalamus, where NPY neurones - which also express the melanocortin receptor antagonist / inverse agonist AgRP (agouti related peptide) - act as the primary "sensory" neurones in the stimulatory branch of the arcuate nucleus. Thus, in this the "sensor nucleus" for the control of appetite and energy expenditure, the NPY/AgRP neurones together with the inhibitory POMC/CART neurones monitor the hormonal and nutritional status of the body as these neurones are the target for both the long-term regulators such as leptin and insulin and short term regulators such as ghrelin and PYY (see below). The stimulatory NPY/AgRP neurones project for example to the paraventricular nucleus - also of the hypothalamus - where its postsynaptic target receptors are believed to be Y1 and Y5 receptors. NPY is the most potent compound known in respect of increasing food intake, as rodents upon intracerebroventricular (ICV) injection of NPY will eat until they literally burst. AgRP from the NPY/AgRP neurones acts as an antagonist mainly on melanocortin receptors type 4 (MC-4) and block the action of POMC derived peptides - mainly aMSH - on this receptor. Since the MC4 receptor signal acts as an inhibitor of food intake, the action of AgRP is - just like the NPY action - a stimulatory signal for food intake (i.e. an inhibition of an inhibition). On the NPY/AGRP neurons are found inhibitory - pre-synaptic - Y2 receptors, which are the target both of locally released NPY as well as a target for the gut hormone PYY - another PP-fold peptide.

**PYY** is released during a meal - in proportion to the calorie content of the meal - from entero-endocrine cells in the distal small intestine and the colon, to act both in the periphery on GI-tract functions and centrally as a satiety signal. Peripherally, PYY is believed to function as an inhibitor - an "illeal break" - on for example upper Gl-tract motility, gastric acid and exocrine pancreatic secretion. Centrally, PYY is believed to act mainly on the presynaptic, inhibitory Y2 receptors on the NPY/AgRP neurones in the arcuate nucleus, which it is beloved to get access to from the blood *(*Batterham et al. 2002 Nature 418: 650-4*).* The peptide is released as PYY1-36, but a fraction - approximately 50 % - circulates as PYY3-36 which is a product of degradation by dipeptidylpeptidase-IV an enzyme which removes a dipeptide from the N-terminus of a peptide provided that a Pro or Ala is found in position two as in all three PP-fold peptides - PP, PYY and NPY *(*Eberlein et al. 1989 Peptides 10: 797-803*).* Thus PYY in the circulation is a mixture of PYY1-36, which acts on both Y1 and Y2 receptors (as well as Y4 and Y5 with various affinities), and PYY3-36 - which has lower affinities for the Y1, Y4 and Y5 receptors than for the Y2 receptor.

**PP** is a hormone, which is released from endocrine cells in the pancreatic islets, almost exclusively governed by vagal cholinergic stimuli elicited by especially food intake *(*Schwartz 1983 Gastroenterology 85:1411-25). PP has various effects on the gastrointestinal tract, but most of these are not observed in isolated cells and organs, and appear to be dependent on an intact vagal nerve supply (Schwartz1983 Gastroenterology 85:1411-25). In accordance with this, the PP receptors, which are called Y4 receptors, are located in the brain stem with a strong expression in vagal motor neurones - activation of which results in the peripheral effects of PP - and in the nucleus tractus solitarirus (NTS) - activation of which results in the effects of PP as a satiety hormone (Whitecomb et al. 1990 Am.J.Physiol. 259: G687-91*,* Larsen & Kristensen 1997 Brain Res.Mol.Brain Res 48: 1-6). It should be noted that PP from the blood has access to this area of the brain since the blood brain barrier is "leaky" in this area where various hormones from the periphery are sensed. Recently it has been argued that part of the effect of PP on food intake is mediated through an action on neurones - especially the POMC/CART neurones in the arcuate nucleus (Batterham et al. 2004 Abstract 3.3 International NPY Symposium in Coimbra, Portugal). PP acts through Y4 receptors for which it has a subnanomolar affinity as opposed to PYY and NPY which have nanomolar affinity for this receptor (Michel et al. 1998 Pharmacol. Rev. 50: 143-150). PP also has an appreciable affinity for the Y5 receptor, but it is not likely of physiological importance in relation to circulating PP due to both lack of access to the cells in the CNS where this receptor especially is expressed and due to the relatively low affinity for PP.

### PP-fold peptide receptors

There are four well established types of PP-fold peptide receptors in man: Y1, Y2, Y4, and Y5 which all recognize NPY1-36 and PYY1-36 with similar affinity. At one time a Y3 receptor type, which might prefer NPY over PYY, was suggested, but today this is not accepted as a real receptor subtype (Michel et al. 1998 Pharmacol. Rev. 50: 143-150). A Y6 receptor subtype has been cloned, however in man this is expressed in a truncated form lacking TM-VII as well as the receptor tail and consequently at least on its own does not appear to form a functional receptor molecule.

**Y1 receptors** - affinity studies suggest Y1 binds NPY and PYY equally well and basically not PP. Affinity for Y1 is dependent on the identities of both end sequences of the PP-fold molecule (NPY/PYY) - for example residues Tyr1 and Pro2 are essential - and on the peptide ends being presented in just the right way. In the C-terminal end, where the side-chains of several of the residues are essential, the Y1 receptor - like the Y4 and Y5 receptor but not the Y2 receptor - tolerates certain substitutions in position 34 (normally a Gln) - such as Pro (Fuhlendorff et al. 1990 J.Biol.Chem. 265: 11706-12*,* Schwartz et al. 1990 Annals NY Acad.Sci. 61: 35-47). Some structure-function studies concerning the requirements of the Y1 and Y2 receptors have been reported *(*Beck-Sickinger et al. 1994 Eur.J.Biochem. 225: 947-58*;* Beck-Sickinger and Jung 1995 Biopolymers 37: 123-42*;* Söll et al. 2001 Eur.J.Biochem. 268: 2828-37).

**Y2 receptors** - affinity studies suggest Y2 binds NPY and PYY equally well and basically not PP. The receptor requires especially the C-terminal end of the PP-fold peptide (NPY/PYY). Thus, long C-terminal fragments - down to for example NPY13-36 (the whole alpha helix plus the C-terminal hexapeptide) - are recognized with relatively high affinity, i.e. to within ten-fold of the affinity of the full-length peptide (Sheikh et al. 1989 FEBS Lett. 245: 209-14*,* Sheikh et al. 1989 J.Biol.Chem. 264: 6648-54). Therefore various N-terminal deletions, which eliminate the binding to the Y1 receptor, still preserve some degree of binding to the Y2 receptor. However, the affinity of the C-terminal fragments is reduced-approximately 10 fold as compared to NPY / PYY for even relatively long fragments. The Gln residue in position 34 of NPY and PYY is highly important for the ligand recognition of the Y2 receptor (Schwartz et al. 1990 Annals NY Acad.Sci. 611: 35-47).

**Y4 receptors -** affinity studies suggest that Y4 binds PP with subnanomolar affinity corresponding to the concentrations found in plasma whereas NPY and PYY are recognized with much lower affinity. Such studies suggest the Y4 receptor is highly dependent on the C-terminal end of the PP-fold peptides, and that relatively short N-terminal deletions impairs the affinities of the ligands. Some structure activity studies concerning the Y4 receptor have been reported *(*Gehlert et al. 1996 Mol.Pharmacol. 50: 112-18*,* Walker et al. 1997 Peptides 18: 609-12*).*

**Y5 receptors -** affinity studies suggest that Y5 binds NPY and PYY equally well, and also binds PP with lower affinity, which however is below the normal circulating levels of this hormone. PYY3-36 is also recognized well by the Y5 receptor, however this receptor is to a large degree expressed in the CNS where such peptide cannot get access to the receptor readily when administered in the periphery.

PP-fold peptides and analogs of these have been suggested for use in the treatment of obesity and associated diseases, including for example Prader Willi's syndrome, based on the demonstrated effects of certain of the these peptides in animal models and in man and on the fact that obese people have low basal levels of PYY and PP as well as lower meal responses of these peptides (Holst JJ et al. 1983 Int.J.Obes. 7: 529-38*;* Batterham et al. 1990 Nature). Infusion of PP in patients with Prader Willi's syndrome was early on shown to decrease food intake (Berntson et al. 1993 Peptides 14: 497-503) and this effect has been confirmed by infusion of PP in normal human subjects (Batterham et al 2003, Clin.Endocrinol.Metab. 88: 3989-92). PP-fold peptides have also been suggested for the use in for example therapeutic angiogenesis (Zukowska et al. 2003 Trends Cardiovasc Med. 13:86-92) and in inflammatory bowl disease (see for example WO 03/105763).

However, the native PP-fold peptides are not optimal for use as biopharmaceuticals. For example, the full length peptides, PYY1-36 and NPY1-36 react too broadly with all Y receptor types and will therefore cause cardiovascular side effects and, for example, emesis. Moreover, the natural peptides are not optimized for protein stability as they are made to normally act for a relatively short time as a neuropeptide or hormone. The naturally occurring, more Y2 selective peptide, PYY3-36 has for example the draw back that its PP-fold structure is impaired due to the elimination of the important Pro2 of the poly-proline helix, which in the full length peptide interacts with Tyr27 in the amphiphilic helical region of the molecule.

For the treatment of conditions responsive to Y receptor modulation, it would therefore be desirable to use Y receptor PP-fold peptides or PP-fold peptide mimics which were specific for the selected Y receptor intended as target, and which stably preserve elements of the PP-fold structure important for receptor binding. In particular, it would be highly desirable to use such agents which are selective for the Y2 receptor over the Y1 and Y4 receptors. The Y2 receptor is the receptor, which will give the beneficial effect on for example food intake and energy expenditure for the treatment of obesity, metabolic syndrome etc. and it is also the Y2 receptor which will give the beneficial effect to obtain therapeutic angiogenesis in patients with for example peripheral vascular disease or coronary vascular disease. However, an agent which acts as a Y2 receptor agonist is not particularly useful for such treatment unless it is selective for the Y2 receptor over the Y1 and the Y4 receptors. Agonism on the Y1 receptor will, for example induce serious side effects in the cardiovascular system - increase in blood pressure - as well as renal system - natriuresis. Similarly, Y2 selectivity over the Y4 receptor is desirable, since the two natural Y2 and Y4 agonists, PYY and PP respectively, have many similar effects on for example the gastrointestinal tract - some of which could be beneficial - but some of which may cause unwanted side effects. For example, both Y2 and Y4 receptors promote anti-secretory effects in the small and large intestine through respectively a neuronal and a direct epithelial mode of action (Cox et al. 2002 Br.J.Pharmacol. 135: 1505-12*).* Thus, it is likely that an additive or even possibly a synergistic anti-secretory effect would be obtained through a combined stimulation of the Y2 and the Y4 receptor, which could lead to constipation.

### Some Common Terms Used In this Specification

**Affinity:** The affinity of a peptide to a specific receptor is given for example as an IC₅₀ value or a Kᵢ or K_{d} value, which in a specific, non-limiting example is determined in an assay, such as a competition binding assay. The IC50 value corresponds to the concentration of the peptide which displaces a - for the given receptor relevant - radioactive ligand used in an amount far less than the Kd for that radioactive ligand to 50%.

**Appetite:** A natural desire, or longing for food. Increased appetite generally leads to increased feeding behavior.

**Appetite Suppressants:** Compounds that decrease the desire for food

**Binding:** A specific interaction between two molecules, such that the two molecules interact. Binding to a receptor can be specific and selective, so that one molecule is bound preferentially when compared to another molecule. Specific binding may be identified by a disassociation constant (K_{d}). This value is dependent on the selectivity of the compound tested. For example, a compound with a K_{d} that is less than 10 nM is generally considered an excellent drug candidate. However, a compound that has a lower affinity, but is selective for the particular receptor, can also be a good drug candidate.

**Body Mass Index (BMI):** A mathematical formula for measuring body mass, also sometimes called Quetelet's Index. BMI is calculated by dividing weight (in kg) by height² (in meters). The current standards for both men and women accepted as "normal" are a BMI of about 20 kg/m². In one embodiment, a BMI of greater than 25 kg/m² can be used to identify an obese subject. Grade I obesity corresponds to a BMI of 25 kg/m². Grade II obesity corresponds to a BMI of 30-40 kg/m²; and Grade III obesity corresponds to a BMI greater than 40 kg/m² *(*Jequier 1987 Ain. J Clin. Nutr. 45:1035-47). Ideal body weight will vary among species and individuals based on height, body build, bone structure, and sex.

**Caloric intake** or **calorie intake:** The number of calories (energy) consumed by an individual. In the present context this term is identical to the term "energy intake".

**Cosmetic treatment:** The term is intended to denote a treatment that is not for medical purposes, but for improving the well-being of a subject e.g. with respect to the appearance of a subject. Included in the term is treatment of a subject who desires to decrease his weight without necessarily being overweight or obese.

**Food intake:** The amount of food consumed by an individual. Food intake can be measured by volume or by weight. Included within its meaning is i) food intake as being the total amount of food consumed by an individual, and ii) food intake is the amount of proteins, fat, carbohydrates, cholesterol, vitamins, minerals, or any other food component, of the individual. Accordingly, the term food intake as used in the present context is similar to the term "energy intake".

**Normal Daily Diet:** The average food intake for an individual of a given species. A normal daily diet can be expressed in terms of caloric intake, protein intake, carbohydrate intake, and/or fat intake. A normal daily diet in humans generally comprises the following: about 2,000, about 2,400, or about 2,800 to significantly more calories. In addition, a normal daily diet in humans generally includes about 12 g to about 45 g of protein, about 120 g to about 610 g of carbohydrate, and about 11 g to about 90 g of fat. A low calorie diet would be no more than about 85%, and preferably no more than about 70%, of the normal caloric intake of a human individual. In animals, the caloric and nutrient requirements vary depending on the species and size of the animal. For example, in cats, the total caloric intake per kg, as well as the percent distribution of protein, carbohydrate and fat varies with the age of the cat and the reproductive state.

**Obesity:** A condition in which excess body fat may put a person at health risk (see Barlow and Dietz, Pediatrics 102:E29, 1998*; National Institutes of Health, National Heart, Lung, and Blood Institute (NHL81),* Obes. Res. 6 (suppl. 2):51 S209S, 1998). Excess body fat is a result of an imbalance of energy intake and energy expenditure. In one embodiment, the Body Mass Index (BMI) is used to assess obesity. In one embodiment, a BMI of from about 22 kg/m² (i.e. about 10% above the normal value) and up to about 30 kg/m² is regarded as overweight, especially from about 25.0 kg/m² and up to 30 kg/m², while a BMI of 30 kg/m² or more is obese.

**Overweight:** An individual who weighs more than their ideal body weight. An overweight individual can be obese, but is not necessarily obese. In one embodiment, an overweight individual is any individual who desires to decrease their weight. In another embodiment, overweight individual is an individual with a BMI of a BMI of from about 22 kg/m² (i.e. about 10% above the normal value) and up to about 30 kg/m² is regarded as overweight, especially from about 25.0 kg/m² and up to 30 kg/m². It should be noted that subjects having a BMI that is only slightly higher than that of the normal value (e.g. from about 22 to about 25 kg/m²) very often have a desire to loose weight although this may only be for cosmetic reasons.

**Potency:** In vitro potency of a compound is defined in terms of EC₅₀ values, i.e. the concentration that leads to 50% of the maximally achievable effect as determined in a for the given receptor relevant signaling assay.

**Subject:** The subject can be any subject, including both human and veterinary mammalian subjects. Thus, the subject can be a human, or can be a non-human primate, a farm animal such as swine, cattle, sheep and poultry, a sport animal or pet such as dogs, cats, horses, hamsters, and rodents.

**Therapeutically effective amount:** A dose sufficient to prevent, treat or ameliorate a specific condition or disease and/or to alleviate specific signs or symptoms of a specific condition or disease. The term includes a dose that is sufficient or prevent advancement, or to cause regression of a disorder, or which is capable of relieving a sign or symptom of a disorder, or which is capable of achieving a desired result. In embodiments relating to cosmetic treatment or to treatment of overweight or obesity, a therapeutically effect of a receptor agonist is an amount sufficient to inhibit or halt weight gain, or an amount sufficient to decrease appetite, or an amount sufficient to reduce energy or food intake or increase energy expenditure. The term "cosmetically effective amount" is intended to denote a dose that is sufficient for the subject being treated to achieve a desired effect.

### DETAILED DESCRIPTION OF THE INVENTION

In its broadest aspect, the present invention provides Y receptor agonist selective for the Y2 receptor over the Y1 and Y4 receptors, selected from the group consisting of:
[Lys4,Leu17,Gln34]PP (SEQ ID No: 23)
[[Cys2, D-Cys27]PYY2-36 (SEQ ID No: 36)
Lys4,Leu17,Leu30.Gln34]PP (SEQ ID No: 24)
[Lys4,Nle17,Nle30,Gln34]PP (SEQ ID No: 25)
[Lys4,Gln34]PP (SEQ ID No: 12)
[Cys2,D-Cys27]PYY (SEQ ID No: 13)
[Cys2,D-Cys27]NPY (SEQ ID No: 14)
[Cys2,Ile3,D-Cys27,Val31]NPY (SEQ ID No: 15)
[Cys2,Ile3,D-Cys27,Leu28,Val31]NPY (SEQ ID No: 32)
[Cys2,Ile3,Nle17,D-Cys27,Nle28,Val31]NPY (SEQ ID No: 33)
and N-acylated and conservatively substituted analogues thereof.

### Specific Invention-Related Terminology

The agonists with which this invention are concerned are **selective for the Y2 receptor over the Y1 and Y4 receptors.** In the present context, this condition is fulfilled if the agonist has an IC50 value that is at least 10-fold lower for the Y2 than for the Y1 and Y4 receptors when measured in the affinity assay described herein. In general, with respect to potency, the agonists of the invention also have EC50 values at least 10-fold lower for the Y2 than for the Y1 and Y4 receptors when measured in the potency assay described herein. Many of the preferred agonists of the invention have affinities and potencies at least 1 00-fold higher for the Y2 receptor than for the Y1 and Y4 receptors, which is a bigger difference than obtained with the natural peptide PYY3-36. Some of the preferred agonists of the invention have affinities and potencies at least 1 000-fold higher for the Y2 receptor than for the Y1 and Y4 receptors.

When the agonists with which the invention is concerned have C- or N- terminal amino acid sequences, the C-termini may be amidated and/or the N-termini may be acylated, to confer resistance to carboxy- and/or aminopeptidases. In fact, the C-terminus of the native NPY, PYY and PP peptides are amidated, so a C-terminal amino acid of an agonist of the invention may also be amidated.

In this specification, reference is made to amino acids by their common names or abbreviations, such as valine (Val), leucine (Leu), isoleucine (Ile), methionine (Met), phenylalanine (Phe), asparagine (Asn), glutamic acid (Glu), glutamine (Gln), histidine (His), lysine (Lys), arginine (Arg), aspartic acid (Asp), glycine (Gly), alanine (Ala), serine (Ser), threonine (Thr), tyrosine (Tyr), tryptophane (Trp), cysteine (Cys) and proline (Pro). When referred to by its common name or abbreviation, without specifying its steroisomeric form, the amino acid in question is to be understood as the L-form. Where the D-form is intended, the amino acid will be specifically referred to as such. Occasionally, where the context makes it desirable to do so, the L-form will be specified rather than inferred.

The term "conservative substitution" as used herein denotes that one or more amino acids is replaced by another, biologically similar residue. Examples include substitution of amino acid residues with similar characteristics, e.g. small amino acids, acidic amino acids, polar amino acids, basic amino acids, hydrophobic amino acids and aromatic amino acids. Non-limiting examples of conservative amino acid substitutions suitable for use in the present invention include those in the following Table and analogous substitutions of the original residue by non-natural alpha amino acids which have similar characteristics. For example, in a preferred embodiment of the invention Met residues are substituted with norleucine (Nle) which is a bioisostere for Met, but which - as opposed to Met - is not readily oxidised. Another example of a conservative substitution with a residue normally not found in endogenous, mammalian peptides and proteins would be the conservative substitution of Arg or Lys with for example, ornithine, canavanine, aminoethylcysteine or other basic amino acid. For further information concerning phenotypically silent substitutions in peptides and proteins, see, for example, Bowie et.al. Science 247, 1306-1310, 1990*.*

| Original residue | Conservative substitution |
|---|---|
| Ala | Gly |
| Arg | Lys |
| Asn | Gln, His, Thr |
| Asp | Glu |
| Gln | Asn, His |
| Glu | Asp |
| His | Asn, Gln |
| Ile | Leu, Val |
| Leu | Ile, Val |
| Lys | Arg |
| Met | Leu, Ile |
| Phe | Tyr, Trp, His |
| Ser | Thr, Asn |
| Thr | Ser, Asn, Gln |
| Trp | Tyr, Phe, His |
| Tyr | Trp, Phe, His |
| Val | Ile, Leu |

Unless the context dictates otherwise, references herein to NPY, PYY and PP peptides and their sequences relate to the human forms of those peptides and their sequences. However, other mammalian NPY, PYY and PP peptides will often constitute PP-fold peptide mimics of human NPY, PYY and PP, or conservatively substituted human NPY, PYY or PP, as those terms are used herein.

### N-terminus

All three types of Y-2 selective agonists with which the invention is concerned may be acylated at their N-terminus to confer resistance to aminopeptidase activity. For example acylation may be with a carbon chain having from 2 to 24 carbon atoms, and N-terminal acetylation is a particular example.

### Adapted agonists for use in accordance with the invention

Up to this point, basic Y2-selective agonists for use in accordance with the invention have been described generally, and specific examples of such agonists have been provided. However, various modifications may be made to such agonists, including the specifically identified agonists, for the purpose of improving their pharmacokinetics, pharmacodynamics and metabolic properties. Such modifications may involve linking the agonist to functional groupings (also known as motifs) known per se in the art of peptidic or proteinaceous pharmaceuticals. Three particular modifications of particular benefit in the case of the agonists with which the invention is concerned, whether of type (a), (b) or (c) are linkage with serum albumin binding motifs, or a glycosaminoglycan (GAG) binding motifs, or PEGylation.

### Serum-albumin binding motifs

Serum albumin binding motifs are typically lipophilic groups, incorporated to enable a prolonged residence in the body upon administration or for other reasons, which may be coupled in various known ways to peptidic or proteinaceous molecules, for example i) via a covalent linkage to e.g. a functional group present on a side-chain amino acid residue, ii) via a functional group inserted in the peptide or in a suitable derivatized peptide, iii) as an integrated part of the peptide. For example, WO 96/29344 (Novo Nordisk A/S) and P. Kurtzhals et al. 1995 Biochemical J. 312: 725-31 and L.B.Knudsen et al. 2000 J.Med.Chem. 43: 1664-69, describe a number of suitable lipophilic modifications which can be employed in the case of the agonists with which this invention is concerned.

Suitable lipophilic groups include optionally substituted, saturated or unsaturated, straight or branched hydrocarbon groups of from 10 to 24 carbon atoms. Such groups may form, or may form part of, a side chain to the backbone of the agonist, for example by ether, thioether, amino, ester or amide linkage to a side chain of an amino acid residue in the backbone, or to a backbone carbon or a branch from a backbone carbon of a non-peptidic linker radical in the backbone of a PP-fold mimic agonist. The chemistry strategy for attachment of the lipophilic group is not critical, but the following side chains including lipophilic groups are examples which can be linked to a backbone carbon of the agonist, or suitable branch therefrom:
CH₃(CH₂)ₙCH(COOH)NH-CO(CH₂)₂CONH- wherein n is an integer from 9 to 15,
CH₃(CH₂)ᵣCO-NHCH(COOH)(CH₂)₂CONH- wherein r is an integer form 9 to 15,
CH₃(CH₂)ₛCO-NHCH((CH₂)₂COOH)CONH- wherein s is an integer from 9 to 15,
CH₃(CH₂)ₘCONH-, wherein m is an integer from 8 to 18,
-NHCOCH((CH₂)₂COOH)NH-CO(CH₂)ₚCH₃, wherein p is an integer from 10 to 16,
-NHCO(CH₂)₂CH(COOH)NH-CO(CH₂)_{q}CH₃, wherein q is an integer from 10 to 16,
CH₃(CH₂)ₙCH(COOH)NHCO-, wherein n is an integer from 9 to 15,
CH₃(CH₂)ₚNHCO-, wherein p is an integer from 10 to 18,
-CONHCH(COOH)(CH₂)₄NH-CO(CH₂)ₘCH₃, wherein m is an integer from 8 to 18,
-CONHCH(COOH)(CH₂)₄NH-COCH((CH₂)₂COOH)NH-CO(CH₂)ₚCH₃, wherein p is an integer from 10 to 16,
-CONHCH(COOH)(CH₂)₄NH-CO(CH₂)₂CH(COOH)NH-CO(CH₂)_{q}CH₃, wherein q is an integer from 10 to 16, and
a partly or completely hydrogenated cyclopentanophenanthrene skeleton.

In one chemical synthetic strategy the lipophilic group-containing side chain is a C₁₂, C₁₄, C₁₆ or C₁₈ acyl group, for example a tetradecanoyl group, acylating an amino group present in the side chain of a residue of the backbone of the agonist.

As stated, the modification of agonists for use in accordance to provide improved serum binding characteristics is a strategy which may be applied in general, and particularly in the case of the specific agonists listed above.

### GAG binding

As in the case of lipophilic serum binding motifs discussed above, the agonists with which this invention are concerned may be modified by incorporation of the GAG binding motif as, or as part of, a side chain to the backbone of the agonist. Known GAG-binding motifs for incorporation in this way include the amino acid sequences XBBXBX and/or XBBBXXBX, wherein B is a basic amino acid residue and X is any amino acid residue. A plurality, for example three, of such sequences may be incorporated in a concatameric (straight chain) or dendrimeric (branched chain) fashion. Specific concatameric GAG motifs include Ala-Arg-Arg-Arg-Ala-Ala-Arg-Ala-Ala-Arg-Arg-Arg-Ala-Ala-Arg-Ala, and Ala-Arg-Arg-Arg-Ala-Ala-Arg-Ala-Ala-Arg-Arg-Arg-Ala-Ala-Arg-Ala-Ala-Arg-Arg-Arg-Ala-Ala-Arg-Ala (both of which may, for example be coupled through an amide bond formed between the C-terminus of the concatameric GAG-binding motif and an amino group in the side chain of a backbone amino acid of the agonist.

Instead of being attached to the agonist as, or as part of a side chain to a backbone residue, the GAG motif may be covalently linked to the C- or (preferably) N-terminus of the agonist, either directly or via a linker radical. Here also the GAG-binding motif may comprise the amino acid sequence XBBXBX and/or XBBBXXBX, wherein B is a basic amino acid residue and X is any amino acid residue, for example the sequence [XBBBXXBX]ₙ where n is 1 to 5, B is a basic amino acid residue and X is any amino acid residue. Such concatameric repeats tend to form alpha helices when they bind to GAG's, and consequently when fused to the C-terminal hexapeptide/last alpha helical turn, can stabilise that turn and thereby present the combined structure in an optimal way for Y2 receptor recognition. Specific examples of agonists of this type are [XBBBXXBX-XBBBXXBX]PYY25-36 or [XBBBXXBX-XBBBXXBX-XBBBXXBX]PYY25-36, wherein B is a basic amino acid residue and X is any amino acid residue.

The Y2 selective agonists with which the present invention is concerned are useful, inter alia, for therapeutic angiogenesis. For this use in particular, the agonists preferably comprise a glycosamino glycan (GAG) binding motif as discussed above. Such motifs ensure that the agonists bind to GAGs in the extracellular matrix, and thereby ensures prolonged local exposure of the Y2 receptors in that tissue. Growth factors, chemokines etc bind to GAGs through patches of basic amino acids, which interact with the acidic sugars of the GAGs. These positively charged epitopes on the growth factors are usually composed of side chains from basic residues, which are not necessarily located consecutively in sequence but are often presented in close proximity by a secondary structural element such as an a-helix or a turn or by the overall three dimensional structure of the protein. Certain GAG-binding, linear sequences, discussed above, have been described, for example XBBXBX and XBBBXXBX where B represents a basic residue (Hileman et al. Bioassays 1998, 20: 156-67). These segments have been shown by circular dichroism to form α-helices upon binding to GAGs. If such sequences are placed for example in a concatameric or dendrimeric construct where for example three such sequences are presented - for example each as a ARRRAARA sequence - the resulting 24-mer peptide - for example ARRRAARA-ARRRAARA-ARRRAARA - ensures a retention in the extracellular matrix similar to high molecular weight polylysine, i.e. it is not washed out during a 4 hour perfusion period (Sakharov et al. FEBS Lett 2003, 27: 6-10).

Thus Growth factors and chemokines are naturally constructed with two types of binding motifs: one binding motif for the receptor through which signal transduction is achieved and one binding motif for GAG's through which attachment, and long-lasting local activity is achieved. Peptides such as PYY and NPY are neuropeptides and hormones, which are rather rapidly washed out of the tissue and are not optimized for long-lasting local activity. By attaching a GAG-binding motif to a Y2 selective agonist according to the present invention or to the classical known peptide agonist PYY3-36 - a bi-functional molecule similar to the growth factors and chemokines is constructed having both a receptor binding epitope in the PP-fold peptide part and a GAG-binding motif. An example of such an agonist is shown in Fig 3A

Another suitable position for introduction of a GAG binding motif - such as a basic concatameric or dendrimeric construct - of the PP-fold peptides is for NPY peptides and analogs thereof, position 14, and for PYY and PP peptides and analogs thereof position 13.

### PEGylation

In PEGylation, a polyalkyleneoxide radical or radicals, is/are covalently coupled to peptidic or proteinaceous drugs to improve effective half life in the body following administration and to reduce immunogenicity, increase solubility etc. The term derives from the preferred polyalkyleneoxide used in such processes, namely that derived from ethylene glycol - polyethyleneglycol, or "PEG".

A suitable PEG radical may be attached to the agonist by any convenient chemistry, for example via a backbone amino acid residue of the agonist. For instance, for a molecule like e.g. PEG, a frequently used attachment group is the epsilon-amino group of lysine or the N-terminal amino group. Other attachment groups include a free carboxylic acid group (e.g. that of the C-terminal amino acid residue or of an aspartic acid or glutamic acid residue), suitably activated carbonyl groups, mercapto groups (e.g. that of a cysteine residue), aromatic acid residues (e.g. Phe, Tyr, Trp), hydroxy groups (e.g. that of Ser, Thr or OH-Lys), guanidine (e.g. Arg), imidazole (e.g. His), and oxidized carbohydrate moieties.

When the agonist is PEGylated it usually comprises from 1 to 5 polyethylene glycol (PEG) molecules such as, e.g. 1, 2 or 3 PEG molecules. Each PEG molecule may have a molecular weight of from about 5 kDa (kiloDalton) to about 100 kDa, such as a molecular weight of from about 10 kDa to about 40 kDa, e.g., about 12 kDa or preferably no more than about 20 kDa.

Suitable PEG molecules are available from Shearwater Polymers, Inc. and Enzon, Inc. and may be selected from SS-PEG, NPC-PEG, aldehyde-PEG, mPEG-SPA, mPEG-SCM, mPEG-BTC, SC-PEG, tresylated mPEG (US 5,880,255), or oxycarbonyl-oxy-N-dicarboxyimide-PEG (US 5,122,614).

### Serum albumin, GAG and PEG

Whether the modification to the agonist is attachment of a group to facilitate serum binding, GAG binding or improved stability via PEGylation, the serum albumin binding motif or GAG binding motif, or PEG radical may be, or may form part of, a side chain of a backbone carbon of the agonist corresponding to any of the following positions of PYY or PP: 1, 3, 4, 6, 7, 10, 11, 12, 13, 15, 16, 17, 18, 19, 21, 22, 23, 25, 26, 28, 29, 30 and 32, or corresponding to any of the following positions of NPY: 1, 3, 4, 6, 7, 10, 11, 12, 14, 15, 16, 17, 18, 19, 21, 22, 23, 25, 26, 28, 29, 30 and 32.

### Conjugation to larger biomolecules

As described above, it is possible to link certain motifs at various positions in the peptide without impairing their high Y receptor affinity (see examples). In a similar way the selective Y2 receptor agonists may be used as fusion proteins where they are linked for example to albumin or another protein or carrier molecule which provides beneficial pharmacokinetic or other types of properties such as for example decreased renal elimination. There are multiple chemical modifications and linkers which can be used for such a covalent attachment as known in the art, just as there are multiple proteins or carriers which can be used. Especially, covalent attachment of the selective Y2 peptide agonist to albumin is preferred and at the positions in the PP-fold structure, which have been pointed out elsewhere herein in relation to modifications with the various motifs. In a preferred embodiment of the invention a peptide terminating in a C-terminal Y2 receptor recognition amino acid sequence is fused to the C-terminal end of a large biomolecule such as albumin. Such fusion proteins can be produced through various semisynthetic techniques where the peptide may be made through peptide synthesis as described herein and the biomolecule through recombinant technology. The fusion protein may also be made enteriely as a recombinant molecule expressed for example as a precursor molecule extended by a Gly-Lys-Arg sequence which when expressed as a secretory protein en eukaryotic cells will be cleaved by biosynthetic enzymes and the Gly turned into the carboxyamide on the C.terminal Tyr residue of the C-terminal Y2 receptor recognition sequence.

### Stabilization

As mentioned at various points herein, many of the of the selective Y2 agonists of the present invention are stabilized in various ways, for example by N-terminal acylation, or by replacement of parts of the agonist backbone with a non-peptidic linker, or by internal cyclizing cross links to stabilize the PP-fold structure. This stabilization in most cases serves two purposes, one being to present the receptor recognition epitopes in an optimal way for the Y2 receptor by preserving the PP-fold; is the other being to stabilize the peptide against degradation, i.e. especially proteolytic degradation. This is particularly important when the selective Y2 agonists are also modified to obtain prolonged half-life, sustained release, and/or long-lasting tissue exposure, by the attachment of the various motifs discussed above. Normally, the peptide agonists will be relatively rapidly eliminated mainly through the kidneys and the protein stability as such may not be a critical issue; however, when the presence of the peptide in various body fluids in one or more ways is prolonged for many hours it is particularly important that the biological activity of the peptide is also kept intact, i.e. that it is stabilized in a form resistant to proteolytic attacks, as described both in general and in relation to the specific examples of selective Y2 agonist peptides, for example the PP-fold stabilized, cyclic [Cys2,D-Cys27]PYY and the various analogs of this.

### Helix Inducing Peptides

Acylation of the N-terminus of the agonists with which the invention is concerned has been mentioned as a means of stabilising the agonist against the action of aminopeptidases. Another stabilising modification involves the covalent attachment of a stabilizing peptide sequence of 4-20 amino acid residues covalently at the N-and/or the C-terminus, preferably the N-terminus. The amino acid residues in such a peptide are selected from the group consisting of Ala, Leu, Ser, Thr, Tyr, Asn, Gln, Asp, Glu, Lys, Arg, His, Met and the like. In an interesting embodiment the N-terminal peptide attachment comprises 4, 5 or 6 Lys residues, for example Lys-Lys-Lys-Lys-Lys-Lys-PYY25-36 (SEQ ID No: 11). These can be linked at the N-terminus of the PP-fold peptide agonist or they may be placed at the N-terminus of an N-terminally truncated PP-fold mimic agonist or an N-terminally shortened PP-fold mimic agonist, where a spacer peptide has been introduced between the new N-terminus an the stabilizing peptide. Such modifications are particularly interesting since a string of Lys residues will have a tendency to form an alpha helical structure and induce this in the subsequent residues, which as discussed above is beneficial for the receptor presentation of the last helical turn and the C-terminal Y2 receptor recognition amino acid sequence of the selective Y2 agonist peptides of the present invention. A general description of such stabilizing peptide extensions is given in WO 99/46283 (Zealand Pharmaceuticals), which is hereby incorporated by reference.

The receptor agonists with which the invention is concerned may be prepared by well-known methods such as, e.g., a synthetic, semisynthetic and/or recombinant method. The methods include standard peptide preparation techniques such as, e.g., solution synthesis, and solid-phase synthesis. Based on textbook and general knowledge within the field, a person skilled in the art knows how to proceed in order to obtain the agonists and derivatives or modifications thereof.

### Specific agonists for use in accordance with the invention

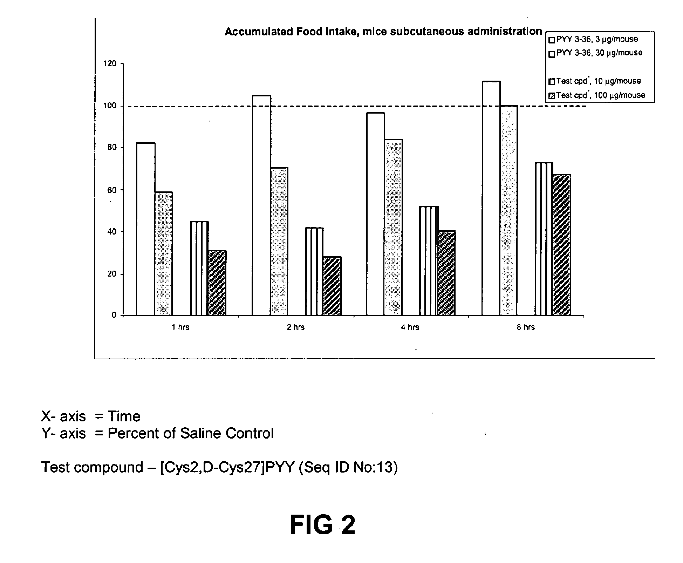

Particularly preferred Y2-selective agonists for use in accordance with the invention are [Cys2,D-Cys27]PYY (SEQ ID No: 13) and [Lys4,Gln34]PP (SEQ ID No: 12; Fig 2B), and conservatively substituted analogues thereof.

In the following, the specific agonists according to the invention are described in relation to their molecular pharmacological properties as well as peptide chemical and stability properties.

### [Lys4,Gln34]PP (SEQ ID No: 12)

This novel peptide is highly selective Y2 receptor agonist, the basic PP-fold peptide beingi PP with a C-terminal Y2 receptor recognition amino acid sequence, which ensures high affinity for the Y2 and Y4 receptor plus low affinity on the Y1 receptor. In order to obtain Y2 over Y4 selectivity a substitution has here been made in position 4 in the N-terminal poly-proline domain of PP, which destroys Y4 recognition. As discussed above, the Y2 over Y4 selectivity may also be obtained through other substitutions. [Lys4,Gln34]PP has the additional interesting property of having a relatively high affinity also for the Y5 receptor as compared to other Y2 selective ligands as well as PP fold peptides in general. Thus [Lys4,Gln34]PP and analogs of this as for example those presented below could be considered to be combined Y2-Y5 selective agonists.

*Receptor recognition profile -* [Lys4,Gln34]PP has an in vitro potency (Table 2 below) on the Y2 receptor very similar to that of PYY3-36. On the Y1 receptor [Lys4,Gln34]PP has an EC50 window of more than 1 00-fold between the potencies on the Y2 and the Y1 receptors, which is similar to PYY3-36. Similarly, [Lys4,Gln34]PP has an almost 100-fold selectivity window to the Y4 receptor. Thus, [Lys4,Gln34]PP is a highly selective Y2 over Y1 and Y4 agonist built on a PP scaffold. Interestingly, [Lys4,Gln34]PP has a very high potency on the Y5 receptor, as opposed to for example PYY3-36. The potency of [Lys4,Gln34]PP on the Y5 receptor is even higher than that of the most potent endogenous ligands PYY and NPY

*Protein stability-* [Lys4,Gln34]PP has a stable PP-fold similar to PP. In order to improve its properties as a biopharmaceutical and prevent for example degradation by amino-peptidases an N-terminal acetylated form could be prepared as discussed above or the substitutions could be combined with for example a D-Ala1 or D-Ala2 substitutions, which would also further improve the Y2 over Y1 recognition window.

### [Lys4,Leu17,Leu30,Gln34]PP (SEQ ID No: 24), and [Lys4,Nle17,Nle30,Gln34]PP (SEQ ID No: 25)

These are novel highly selective Y2 receptor agonists, the basic PP-fold peptide being PP with a C-terminal Y2 receptor recognition amino acid sequence, which ensures high affinity for the Y2 and Y4 receptor plus low affinity on the Y1 receptor. As for [Lys4,Gln34]PP, in order to obtain Y2 over Y4 selectivity a substitution has here been made in position 4 in the N-terminal poly-proline domain of PP, which destroys Y4 recognition. Like [Lys4,Gln34]PP these peptides have the additional interesting property of having a relatively high affinity also for the Y5 receptor as compared to other Y2 selective ligands as well as PP fold peptides in general. Importantly, in [Lys4,Leu17,Gln34]PP, [Lys4,Leu17,Leu30,Gln34]PP, and [Lys4,Nle17,Nle30,Gln34]PP one or both of the Met residues in position 17 and 30 have been substituted with either a Leu or a norleucine (Nle) which cannot be oxidized. Thus these three peptides here represent a group of Y2 selective peptides in which oxidation of Met residues has been prevented through substitution with a non-oxidizable residue.

*Protein stability-* [Lys4,Leu17,Gln34]PP, [Lys4,Leu17,Leu30,Gln34]PP, and [Lys4,Nle17,Nle30,Gln34]PP [Lys4,Gln34]PP has a general stability similar to that of [Lys4,Gln34]PP, however these peptides are not susceptible to oxidation of one or more of the Met residues, which in certain biopharmaceutical circumstances is an advantage.

### [Cys2,D-Cys27]PYY (SEQ ID No: 13)

This peptide is a highly selective Y2 agonist which has been designed for high protein stability. In analogy with the C-2 NPY and C-2 PYY constructs an intra-molecular disulfide bridge has been introduced between a Cys in positions 2 and a D-Cys in position 27, which however here have been introduced in the full length PYY molecule in order to stabilize its PP-fold structure. Thus, [Cys2,D-Cys27]PYY here represents a group of selective Y2 agonist peptides with a stabilizing intra-molecular bond between the N-terminal poly-Pro sequence and the amphiphilic helical segment. In other members of this group the disulfide bridge could be made between residues at other positions or another type of covalent, intra-molecular link could be established, for example a lactam bridge between the gamma-carboxyl group of Glu2 and the epsilon amino group of Lys30 in for example [Glu2-Lys30]PYY. [Cys2,D-Cys27]PYY is active for example also with the N-terminus acetylated, a modification, which further prevents the degradation of the peptide by aminopeptidases. [Cys2,D-Cys27]PYY is a novel compound.

*Receptor recognition profile-* [Cys2,D-Cys27)PYY stimulates the Y2 receptor (Table 2 below) with a potency (EC50 = 0.33 nM) similar to PYY3-36, whereas it stimulates the Y1 receptor with a potency of more than 1 µM which is more than 20 fold less potent on the Y1 receptor than PYY3-36. On the Y4 receptor [Cys2,D-Cys27]PYY has an EC50 of more than 1 µM. Thus [Cys2,D-Cys27]PYY is a subnanomolar selective Y2 agonist with a selectivity index towards the Y1 receptor of around 10.000, which is 50-100 fold better than PYY3-36

*Protein stability -* due to the PP-fold stabilizing, intra-molecular disulfide bridge [Cys2,D-Cys27]PYY is more stabile than PYY3-36, in which Pro2 which is an important part of the hydrophobic sore which stabilized the PP-fold has been removed, and even more stable than PYY1-36 itself. The disulfide bridge basically prevents the PP-fold from being "unzipped" and the PP-fold holds the peptide in a conformation in which it is difficult for endoproteinases to get access to the peptide. The substitution of Pro2 with Cys had the additional advantage that it renders the peptide totally resistant to degradation by dipeptidylpeptidase IV for which PYY1-36 otherwise is a fairly good substrate This increased stability is even further increased in the N-terminally acetylated version of [Cys2,D-Cys27]PYY.

*In vivo receptor selectivity -* In a dose-escalation study [Cys2,D-Cys27]PYY was administered by intravenous infusion to anesthetized dogs in consecutive 30 minute infusion periods in doses of 0.3, 1, 3, 10, 30 and 100 µg/kg. Plasma levels of [Cys2,D-Cys27]PYY was measured by radioimmunoassay, which demonstrated a linear dose relationship. At the highest dose a plasma levels of 32 nM was obtained in the dogs. Nevertheless no dose-dependent effect on neither blood pressure nor on heart rate was observed. It is well known that PYY and NPY, which are agonists both on the Y1 and the Y2 receptors, at much lower plasma levels will increase blood pressure. Thus these experiments indicate that [Cys2,D-Cys27]PYY also in vivo is a highly selective peptide devoid of any measurable Y1 receptor activity.

*In vivo effect on acute food intake -* Either PYY3-36, [Cys2,D-Cys27]PYY or saline was administered by subcutaneous injections to groups of 8 mice in doses of 3 or 30 µg (PYY3-36) or 10 and 100 µg ([Cys2,D-Cys27]PYY) per animal after 16 hours of fasting. In Fig. 2 is shown the accumulated food intake of the mice. PYY3-36 at a dose of 30 µg inhibited food intake during the first two hours, but the effect gradually disappeared over the following 2 to 6 hours. [Cys2,D-Cys27]PYY had a more pronounced and long-lasting inhibitory effect on food intake in mice than PYY3-36. Thus even at a dose of 10 µg a more efficacious inhibition of food intake was observed and the effect lasted for more than 8 hours Fig 2. Thus [Cys2,D-Cys27]PYY, which is equipotent with PYY3-36 on the Y2 receptor as determined through in vitro signal transduction assays, appeared to be more potent and to have a more prolonged effect on acute food intake in mice in vivo. This effect could possibly be related to the increased protein stability, which is inherent to the disulfide bridge stabilized [Cys2,D-Cys27]PYY peptide.

### N-(N'-tetradecanoyl)-gammaglutamoyl-[Cys2, D-Cys27]PYY (SEQ ID No: 35)

This peptide represent a representative member of a family of peptides which are highly Y2 selective agonists, which are protein chemistry stabilized with an intramolecular disulfide bridge (to prevent enodopeptidase degradation) and which are N-terminally acetylated to prevent aminopeptidase degradation (the peptide is naturally C-terminally amidated which prevents carboxypeptidase degradation) and which are alkylated at the alpha amino group of Tyr1 with a long chain fatty acid, which besides preventing the attack by amino peptideases also in the circulation secures binding of the modified peptide to albumin and thereby provides a long duration of action for the peptide.

### N-acetyl[Cys2-DCys27]PYY2-36 (SEQ ID No: 27) and [Cys2-DCys27]PYY2-36 (SEQ ID No: 36)

The peptide - [Cys2-DCys27]PYY2-36 - is a highly selective Y2 agonist of type (c), which also has been designed for high protein stability. In analogy with [Cys2-DCys27]PYY an intra-molecular disulfide bridge has been introduced between a Cys in positions 2 and a D-Cys in position 27, which however here has been introduced in PYY2-36, in order to stabilize its PP-fold structure. Tyr1 is either absent or has been substituted with an N-terminal acetylation. [Cys2,D-Cys27]PYY2-36 and the N-terminally acetylated form can be modified by various motifs - for example by introduction of an attachment site as a Lys13 or other residue at another position as indicate elsewhere herein - as presented for other Y2 selective peptides at positions which will preserve their Y2 selectivity while adding other beneficial properties as discussed above. [Cys2,D-Cys27]PYY2-36 and the N-terminally acetylated form of this are novel compounds.

N-acetyl[Cys2-DCys27]PYY2-36 has Y1, Y2 and Y4 have receptor recognition properties very similar to those of [Cys2-DCys27]PYY.

### [Cys2,Ile3,D-Cys27,Val31]NPY (SEQ ID No: 32), and [Cys2,Ile3,D-Cys27,Leu28,Val31]NPY (SEQ ID No: 33).

These are all highly selective Y2 agonists of type (c), which in analogy with [Cys2-DCys27]PYY have an intra-molecular disulfide bridge introduced between a Cys in positions 2 and a D-Cys in position 27, which however here have been introduced in NPY, and in the various peptides amino acid residues have been substituted in the C- and/or N-terminal ends in order to make the peptide more PYY like but keep the general PP-fold of NPY. In some of the analogs, Met residues have been substituted with Leu or Nle residues to avoid potential oxidation of the peptide.

### Clinical Indications

The Y2-specific agonists with which the invention is concerned are of value in the treatment of conditions responsive to activation of Y2 receptors. Such conditions include those for which regulation of energy intake or energy metabolism, or induction of angiogenesis, is indicated. For any such use, the agonist may be one which comprises a modification or motif which confers stability towards peptidases, serum protein binding properties, or PEGylation to prolong serum and / or tissue half-life. Especially for induction of angiogenesis, the agonist may comprise a GAG-binding motif to prolong tissue half-life and Y receptor exposure.

Diseases or conditions where induction of angiogenesis is indicated include peripheral vascular disease, coronary vascular disease, myocardial infarction, stroke, conditions in which any of the foregoing is considered a contributory factor, wound healing and tissue repair.

Diseases or conditions in which regulation of energy intake or energy metabolism is indicated include obesity and overweight, and conditions in which obesity and overweight are considered contributory factors, such as bulimia, bulimia nervosa, Syndrome X (metabolic syndrome), diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, coronary artery disease, myocardial infarction, peripheral vascular disease stroke, thromboembolic diseases, hypercholesterolemia, hyperlipidemia, gallbladder disease, osteoarthritis, sleep apnea, reproductive disorders such as polycystic ovarian syndrome, or cancer of the breast, prostate, or colon.

### 1. Obesity and Overweight

PYY3-36 has been shown to decrease appetite, food intake and body weight in various rodents *(*Batterham et al. Nature 2002, 418: 595-7*;* Challis et al. BBRC Nov. 2003, 311: 915-9*)* as well as to decrease appetite and food intake in man *(Batterham et al 2002).* The animal data including studies in receptor knock out animals strongly indicate that this effect of PYY3-36 is mediated through Y2 receptors and through NPY/AgRP and POMC neurones in the arcuate nucleus. Interestingly, the effect is very long lasting and is seen up to 24 hours after for example a single intra-peritoneal injection of PYY3-36. Such long lasting effects on appetite etc, is well know also from ICV injection of especially AgRP. PYY levels and the PYY food responses are lower in obese subjects and correlates inversely with their BMI. Importantly, obese subject are not resistant to the effect of PYY as infusion of PYY3-36 for 90 minutes decreases food intake in obese subjects in a similar long lasting fashion *(*Batterham et al. 2003, NEJM 349: 941-48*).*

Hence, the Y2 selective agonists with which the invention is concerned are suitable for use in a subject, such as a mammal including a human, in order to regulate the energy intake. Accordingly, the invention relates to methods for altering energy intake, food intake, appetite, and energy expenditure. A method is disclosed herein for reducing energy or food intake by administering to a subject a cosmetically or therapeutically effective amount of such an agonist. In one embodiment, administration of the receptor agonist results in a decrease in the amount, either the total weight or the total volume of food. In another embodiment, it may result in a decrease of the intake of a food component, such as a decrease in the ingestion of lipids, carbohydrates, cholesterol, or proteins. In the any of the methods disclosed herein, the preferred compounds that have been discussed in details herein could be administered. In an additional embodiment, a method is disclosed herein for reducing appetite by administering a therapeutically effective amount of such an agonist. Appetite can be measured by any means known to one of skill in the art.

For example, decreased appetite can be assessed by a psychological assessment. In such an embodiment, administration of the receptor agonist results in a change in perceived hunger, satiety, and/or fullness. Hunger can be assessed by any means known to one of skill in the art. In one embodiment, hunger is assessed using psychological assays, such as by an assessment of hunger feelings and sensory perception using e.g. a questionnaire.

In a further embodiment, a method is disclosed herein for altering energy metabolism in a subject. The method includes administering a therapeutically effective amount of such an agonist thereof to the subject, thereby altering energy expenditure. Energy is burned in all physiological processes. The body can alter the rate of energy expenditure directly, by modulating the efficiency of those processes, or changing the number and nature of processes that are occurring. For example, during digestion the body expends energy moving food through the bowel, and digesting food, and within cells, the efficiency of cellular metabolism can be altered to produce more or less heat. In a further embodiment a method is disclosed herein for any and all manipulations of the arcuate circuitry described in this application, that alter food intake coordinately and reciprocally alter energy expenditure. Energy expenditure is a result of cellular metabolism, protein synthesis, metabolic rate, and calorie utilization. Thus, in this embodiment, peripheral administration results in increased energy expenditure, and decreased efficiency of calorie utilization. In one embodiment, a therapeutically effective amount of a receptor agonist according to the invention is administered to a subject, thereby increasing energy expenditure.

In several embodiments both relating ot the therapeutic use and to the cosmetic use, a Y2 selective agonist can be used for weight control and treatment, reduction or prevention of obesity, in particular any one or more of the following: preventing and reducing weight gain; inducing and promoting weight loss; and reducing obesity as measured by the Body Mass Index. As mentioned above, the invention also relates to the use of a Y2 selective agonist for controlling any one or more of appetite, satiety and hunger, in particular any one or more of the following: reducing, suppressing and inhibiting appetite; inducing, increasing, enhancing and promoting satiety and sensations of satiety; and reducing, inhibiting and suppressing hunger and sensations of hunger. The disclosure further relates to the use of a Y2 selective agonist in maintaining any one or more of a desired body weight, a desired Body Mass Index, a desired appearance and good health.

In a further or alternative aspect, the invention relates to a method for the treatment and/or prevention of reduced energy metabolism, feeding disorders, appetite disorders, overweight, obesity, bulimia, bulimia nervosa, Syndrome X (metabolic syndrome), or complications or risks associated thereto including diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, congestive heart failure, stroke, myocardial infarct, thromboembolic diseases, hypercholesterolemia, hyperlipidemia, gallbladder disease, osteoarthritis, sleep apnea, reproductive disorders such as polycystic ovarian syndrome, cancers of the breast, prostate, and colon, the method comprising administering to a subject such as a mammal including a human, an effective dose of one or more of a Y2 selective agonists as described herein.

### 2. Therapeutic angiogenesis

A number of *in vitro* studies on effects on growth of vascular smooth muscle cells, hyperthrophy of ventricular cardiomyocytes as well as endothelial cell proliferation and migration have suggested that NPY may act as an angiogenic factor *(*Zukowska-Grojec et al. 1998 Circ.Res. 83: 187-95*).* Importantly, in vivo studies using both the mouse corneal micropocket model as well as the chick chorioallantoic membrane (CAM) assay has confirmed that NPY is a potent angiogenic factor which gives rise to vascular tree-like structures showing vasodilation as observed otherwise only with fibroblast growth factor-2 (FGF-2) and not for example vascular endothelial growth factor (VEGF) angiogenic structures *(*Ekstrand et al. 2003 PNAS 100: 6033-38*).* In the developing chick embryo NPY induced vascular sprouting from preexisting blood vessels. The effect of NPY was not observed in Y2 receptor knock out animals indicating that the Y2 receptor is responsible for the angiogenic effect of NPY *(Ekstrand et al 2003).* This notion is also supported by observations that the Y2 receptor is highly upregulated in ischemic vessels and the enzyme which generates the endogenous, selective Y2 ligand PYY3-36, dipentidylpeptidase-IV is also highly upregulated.

In various cardiovascular diseases such as atherosclerosis for example in peripheral vessels as well as in coronary vessels is it contemplated that induction or angiogenesis would be beneficial. Also induction of angiogenesis is believed to be beneficial for securing reperfusion after myocardial infarction. Especially FGF-2 has been proposed to be an efficient agent for induction of angiogenesis in patients with cardiovascular diseases. However, like most other angiogenic factors FGF-2 is a growth factor and has the potential of stimulating tumor growth also by providing angiogenesis. As presented above, NPY acting through Y2 receptors induces neovascularization of a similar type as induced by FGF-2, however NPY is a neuropeptide and not a classical growth factor and has not been implicated in inducing tumor growth. Thus, a Y2 agonist is a useful agent for therapeutic angiogenesis. However, it is particularly important for this use that the agonist does not show Y1 receptor agonism because this will give unwanted cardiovascular effects. This means that all the peptides with which the invention is concerned, which are selective Y2 selective receptor agonists or which are especially useful therapeutic agents also in respect of inducing angiogenesis. They are particularly useful when modified to attach a GAG binding motif, as discussed above. To elaborate further: The action of FGF-2 as that of most other classical growth factors is partly mediated or controlled through their binding to glycosaminoglycans (GAG) in the extracellular matrix. This binding to GAGs secures that the angiogenic factor acts in an appropriate spatial and temporal fashion and that it is not washed out of the tissue rapidly. For the use of small peptides and peptide mimics such as the ones described in the present invention in therapeutic angiogenesis this is particularly important. Thus, in a preferred embodiment of the invention the peptides incorporate one or more GAG binding motif, which secures that they attach to GAGs in the extracellular matrix in order to induce optimal angiogenesis after administration. This can for example be by intravenous or intraarterial administration or for example direct administration into the coronary arteries in order to induce cardiac angiogenesis during coronary artery disease and/or post acute myocardial infarction. Similarly such a compound can be administered through intra arterial injection in the femoral artery for treatment of peripheral vascular disease. It can also be for example topical local administration to skin lesions in order to promote improved wound healing. A prolonged Y receptor exposure efficient in inducing angiogenesis can also be obtained by using a peptide according to the present invention modified with a serum albumin binding motif.

Thus in a preferred embodiment of the invention the Y2 selective agonists comprise a GAG-binding motif, which is placed in a position where it does not impair the stability of the peptide or impair the potency and selectivity of the peptide. A specific embodiment of the invention relates to PYY3-36 comprising one or more GAG-binding motifs.

In another embodiment of the invention the pharmaceutical composition comprising a therapeutic efficient amount of the Y2 and or Y4 selective agonists comprising a GAG-binding motif in combination with heparin in a therapeutically effective carrier.

Accordingly, in one embodiment the invention relates to the use a Y2 selective receptor agonist modifying disturbances in the angiogenesis system, especially for inducing angiogenesis such as angiogenesis associated with diseases or conditions such as e.g., cardiovascular diseases including peripheral vascular disease with symptoms such as cladicatio intermittens, coronary artery disease and myocardial infarction; tissue repair processes including wound healing in the skin, inflammatory conditions including inflammatory conditions in the gastrointestinal tract such as, e.g., ulcers, colitis, inflammatory bowel disease, Crohns disease etc.

A specific embodiment is to use the receptor agonist for inducing angiogenesis in a heart or in a blood vessel, or in a tissue such as a mucosal tissue including the gastro-intestinal mucosa and the skin.

### 3. Wound healing

In animals where the Y2 receptor has been selectively eliminated through the deletion of its gene it has been reported that wound healing is impaired and that the associated neo-vascularization is impaired *(*Ekstrand et a/. 2003 PNAS 100: 6033-38*).* Thus the selective Y2 agonists of the present invention are useful to improve wound healing. The peptides can for this indication be administered in various way including parenteral administration. However, a preferred route of administration is topical application e.g. in the form of a solution, dispersion, powders, sticks, creme, ointment, lotion, gel, hydrogel, transdermal delivery system including patches and plasters, etc. For topical administration they can be used as such. However, in a preferred embodiment of the invention, the peptides have been modified with one or more of the GAG-binding motifs described herein to ensure a long lasting, local effect of the peptide through binding to GAGs in the tissue.

### 4. Inflammatory bowel disease

PYY has previously been described for the prevention and/or treatment of inflammatory bowel disease; see WO 03/105763 to Amylin Pharmaceuticals, Inc, which is hereby incorporated by reference. Therefore the agonists with which the invention is concerned are effective in the treatment or prevention of inflammatory bowel disease as well. Accordingly, the present invention also relates to the use of the agonists described herein for such medical use. In an interesting embodiment, the peptides comprise one or more GAG-binding motifs, cf. above.

### 5. Osteoporosis

Several studies in Y2 knock out animals have shown very strong effects on trabecular bone formation *(eg* Sainsbury et al. Mol.Cell.Biol. 2003, 23: 5225-33*).* Y2 receptor is also involved in bone formation, cf. Baldock et al. 2002 J.Clin.lnvest 109: 915-21. Thus the present Y2-selective agonists are useful for the treatment of osteoporosis. Especially, it is contemplated that the peptides comprising one or more GAG-binding motifs are suitable for use in osteoporosis or related diseases.

In a subgroup of the population, Y2 agonists may not have the intended action due to genetic variations such as polymorphisms in the Y2 gene. Loss of function mutations in these receptors are likely to be associated with obesity. Thus, in a preferred embodiment of the invention an analysis of the Y2 gene of the subject to be treated is performed in order to probe for polymorphisms / mutations in these genes and identification of such polymorphisms. Based on such an analysis an optimal treatment of the subjects can be made. For example, only subjects with normal genotype or with polymorphisms, which do not affect the function of Y2 agonists, should be treated with such agonists. Another possibility is to increase the dose of the Y2 agonist in subjects who express an impaired receptor in order to ensure an optimal effect of the drug. In the case where the obesity of a subject is caused by an impairment in the function of the Y2 receptor it could be argued that treatment with a - for example large doses - of a Y2 agonist is a form of replacement therapy - provided that at least some of the relevant receptor function is still left - for example in heterozygote patients.

In one embodiment of the invention an acute test may be performed where a Y2 agonist is administered to ensure that these compounds have the intended effect in the subject to be treated before a chronic treatment is started. Through these means it is ensured that only subjects who are susceptible to treatment with Y2 agonists are treated with these compounds.

### Use of Y2 selective agonists in combination with Y4-selective agonists and other agents

Our copending International patent application filed on even date herewith discloses Y4-selective agonists and their use in treatment. The Appendix to this application briefly summarises the structural characteristics required of a Y4 agonist according to that application, and gives specific examples of Y4 selective agonists. As briefly discussed below, the Y2-agonists of this invention may be used in treatment together with Y4 specific agonists.

Through combined treatment with two compounds, respectively a selective Y2 agonist and a selectiveY4 agonist it is possible to dose each of these compounds freely i.e. independently of each other in order to obtain a maximal beneficial effect. That is, it is for example possible to administer a sub-maximal dose of the selective Y2 compound in order to perhaps ensure that only minimal side effects are obtained through for example peripheral cardiovascular Y2 receptors or generation of emesis, while at the same time administer a maximal or even supra-maximal dose of a Y4 agonist to ensure a very strong effect in all subjects through the Y4 receptor pathway, which has a broader therapeutic window. The concomitant - partial - stimulation of the Y2 receptor pathway will ensure a more efficient effect on appetite regulation and energy expenditure than the Y4 agonist stimulation alone. For example, the Y2 agonist may have a more pronounced effect on gastric emptying than the Y4 agonist. And, the concomitant stimulation of the Y4 receptor will allow for the use of a lower and safer dose of the Y2 agonist, which alone would not have been efficient.

In those cases where two individual compounds are used to obtain a combined stimulation of the Y2 and Y4 receptors, the administration regimen may be adjusted as mentioned above. Accordingly, in specific embodiments of the invention, a combination of a Y2 agonist and a Y4 agonist is administered so that the ratio between the dose of Y2 and the dose of Y4 is from about 0.1:10 to about 10:0.1 such as, e.g. from about 0.1:1 to about 1:0.1, from about 0.2:1 to about 1:0., from about 0.3:1 to about 1:0.3, from about 0.4:1 to about 1:0.4 or from about 0.5:1 to about 1:0.5 such as, e.g. 1:1, 1:2, 1:3, 1:4, 1:5, 1:6 etc. As mentioned above, an interesting embodiment is a combination of a Y2 and a Y4 agonist, wherein the balance between the amount of Y2 relative to Y4 is adjusted so that maximal effect is achieved with a minimum (if any) side effects. Accordingly, it is envisaged that the concentration of the Y2 agonist in such a combination is less than the concentration of the Y4 agonist. To this end, the concentration and the ratios mentioned above relates to the molar concentration and the molar ratio, respectively.

Thus in a preferred embodiment of the invention a selective Y2 agonist is administered in conjunction with a selective Y4 agonist for the treatment of obesity.

The compounds can be administered through the same administration route or through different administration routes. The administration route can in principle be any route such as those mentioned herein, the parenteral and the topical route being of most interest. Accordingly, in one embodiment of the invention the Y2 and Y4 agonists are both administered by e.g. the topical route such as by nasal administration or by subcutaneous injection and in another embodiment, the Y2 agonist is administered by nasal administration and the Y4 agonist is administered by subcutaneous injection or vice versa. Other suitable combinations or administration routes are within the scope of the present invention.

The Y2 and Y4 agonists used in a combination treatment may be contained in the same pharmaceutical composition (e.g. in admixture in a pharmaceutical composition) or they may be presented in separate pharmaceutical compositions for simultaneous, sequential or individualized administration with specific instructions for use. Accordingly, such compositions may be presented in the form of a kit comprising a Y2 agonist and a Y4 agonist in the same or in individual containers and, optionally with instructions for use.

The selective Y2 agonists of the invention may be combined in the treatment of obesity, diabetes and related diseases with the use of various other drugs targeting appetite and energy expenditure, this includes but is not limited to drugs such as Gl-tract lipase inhibitors, neurotransmitter reuptake inhibitors, cannabinoid receptors antagonists and inverse agonists, as well as other types of neurotransmitter - including but not limited to 5HT receptors - and/or hormone - including but not limited to GLP-1, MC4, MC3 - receptor agonist or antagonists. Due to the fact that the selective Y2 agonists are targeting a homeostatic regulatory mechanism in the communication between the GI-tract and the CNS - i.e. the Y2 receptors normally targeted by the satiety mediating hormone PYY from the gut - it is particularly beneficial to combine the treatment with the combined Y2 selective agonists with the treatment with a drug targeting a central, hedonic mechanism in the regulation of appetite and energy expenditure, such as the CB1 receptors, for example being part of the reward system. Thus, the use of selective Y2 agonists in the treatment of obesity and related diseases in combination with a CB1 antagonist is a preferred embodiment of this invention.

### Dosages

The therapeutically effective amount of a Y2 receptor agonist according to the invention will be dependent on specific agonist employed, the age, weight and condition of subject being treated, the severity and type of the condition or disease being treated, the manner of administration and the strength of the composition applied.

For example, a therapeutically effective amount of a Y2 receptor agonist thereof can vary from about 0.01 µg per kilogram (kg) body weight to about 1 g per kg body weight, such as about 1 µg to about 5 mg per kg body weight, or about 5 µg to about 1 mg per kg body weight. In another embodiment, the receptor agonist is administered to a subject at 0.5 to 135 picomole (pmol) per kg body weight, or about 72 pmol per kg body weight.

In one specific, non-limiting example from about 5 to about 50 nmol is administered as a subcutaneous injection, such as from about 2 to about 20 nmol, or about 1.0 nmol is administered as a subcutaneous injection. The exact dose is readily determined by one skilled in the art based on the potency of the specific compound (such as the receptor agonist) utilized, the age, weight, sex and physiological condition of the subject. The dose of an agonist can be a molar equivalent of the therapeutically effective dose of PYY3-36.

The amounts can be divided into one or several doses for administration daily, every second day, weekly, every two weeks, monthly or with any other suitable frequency. Normally, the administration is once or twice daily.

### Methods of administration

The Y2 receptor agonist as well as cosmetic or pharmaceutical compositions according to the invention can be administered by any route, including the enteral (e.g. oral administration) or parenteral route. In a specific embodiment, the parenteral route is preferred and includes intravenous, intraarticular, intraperitoneal, subcutaneous, intramuscular, intrasternal injection and infusion as well as administration by the sublingual, transdermal, topical, transmucosal including nasal route, or by inhalation such as, e.g., pulmonary inhalation. In specific embodiments, the subcutaneous and/or the nasal administration route is preferred.

The receptor agonists can be administered as such dispersed in a suitable vehicle or they can be administered in the form of a suitable pharmaceutical or cosmetic composition. Such compositions are also within the scope of the invention. In the following are described suitable pharmaceutical compositions. A person skilled in the art will know how that such composition may also be suitable for cosmetic use or he will know how to adjust the compositions to cosmetic compositions by use of suitable cosmetically acceptable excipients.

### Pharmaceutical compositions

The receptor agonists (also denoted "compounds") according to the invention for use in medicine or cosmetics are normally presented in the form of a pharmaceutical composition comprising the specific compound or a derivative thereof together with one or more physiologically or pharmaceutically acceptable excipients.

The compounds may be administered to an animal including a mammal such as, e.g., a human by any convenient administration route such as, e.g., the oral, buccal, nasal, ocular, pulmonary, topical, transdermal, vaginal, rectal, ocular, parenteral (including inter alia subcutaneous, intramuscular, and intravenous cf. above), route in a dose that is effective for the individual purposes. A person skilled in the art will know how to chose a suitable administration route. As mentioned above, the parenteral administration route is preferred. In a specific embodiment, the receptor agonists are administered subcutaneously and/or nasally. It is well known in the art that subcutaneous injections can be easily self-administered.

A composition suitable for a specific administration route is easily determined by a medical practitioner for each patient individually. Various pharmaceutically acceptable carriers and their formulation are described in standard formulation treatises, e.g., Remington's Pharmaceutical Sciences by E. W. Martin.

The pharmaceutical composition comprising a compound according to the invention may be in the form of a solid, semi-solid or fluid composition. For parenteral use, the composition is normally in the form of a fluid composition or in the form of a semi-solid or solid form for implantation.

Fluid compositions, which are sterile solutions or dispersions can utilized by for example intravenous, intramuscular, intrathecal, epidural, intraperitoneal or subcutaneous injection of infusion. The compounds may also be prepared as a sterile solid composition, which may be dissolved or dispersed before or at the time of administration using e.g. sterile water, saline or other appropriate sterile injectable medium.

The fluid form of the composition may be a solution, an emulsion including nano-emulsions, a suspension, a dispersion, a liposomal composition, a mixture, a spray, or a aerosol (the two latter types are especially relevant for nasal administration).

Suitable mediums for solutions or dispersions are normally based on water or pharmaceutically acceptable solvents e.g. like an oil (e.g. sesame or peanut oil) or an organic solvent like e.g. propanol or isopropanol. A composition according to the invention may comprise further pharmaceutically acceptable excipients such as, e.g., pH adjusting agents, osmotically active agents e.g. in order to adjust the isotonicity of the composition to physiologically acceptable levels, viscosity adjusting agents, suspending agents, emulsifiers, stabilizers, preservatives, antioxidants etc. A preferred medium is water.

Compositions for nasal administration may also contain suitable non-irritating vehicles such as, e.g., polyethylene glycols, glycofurol, etc. as well as absorption enhancers well known by a person skilled in the art (e.g. with reference to Remington's Pharmaceutical Science)

For parenteral administration, in one embodiment the receptor agonists can be formulated generally by mixing it at the desired degree of purity, in a unit dosage injectable form (solution, suspension, or emulsion), with a pharmaceutically acceptable excipient or carrier, i.e., one that is non-toxic to recipients at the dosages and concentrations employed and is compatible with other ingredients of the composition.

Generally, the formulations are prepared by contacting the receptor agonist uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes. Due to the amphiphatic nature of the peptides described herein suitable forms also include micellar formulations, liposomes and other types of formulations comprising one or more suitable lipids such as, e.g., phospholipids and the like.

Preferably, they are suspended in an aqueous carrier, for example, in an isotonic buffer solution at a pH of about 3.0 to about 8.0, preferably at a pH of about 3.5 to about 7.4, 3.5 to 6.0, or 3.5 to about 5. Useful buffer substances include acetate, citrate, phosphate, borate, carbonate such as, e.g., sodium citrate-citric acid and sodium phosphate-phosphoric acid, and sodium acetate/acetic acid buffers.

The compositions may also be designed to controlled or prolonged delivery of the receptor agonist after administration in order to obtain a less frequent administration regimen. Normally a dosage regimen including 1-2 daily administrations is considered suitable, but within the scope of the present invention is also included other administration regimens such as, e.g., more frequent and less frequent. In order to achieve a prolonged delivery of the receptor agonist, a suitable vehicle including e.g. lipids or oils may be employed in order to form a depot at the administration site from which the receptor agonist is slowly released into the circulatory system, or an implant may be used. Suitable compositions in this respect include liposomes and biodegradable particles into which the receptor agonist has been incorporated.

In those situations where solid compositions are required, the solid composition may be in the form of tablets such as, e.g. conventional tablets, effervescent tablets, coated tablets, melt tablets or sublingual tablets, pellets, powders, granules, granulates, particulate material, solid dispersions or solid solutions.

A semi-solid form of the composition may be a chewing gum, an ointment, a cream, a liniment, a paste, a gel or a hydrogel.

Other suitable dosages forms of the pharmaceutical compositions according to the invention may be vagitories, suppositories, plasters, patches, tablets, capsules, sachets, troches, devices etc.

The dosage form may be designed to release the compound freely or in a controlled manner e.g. with respect to tablets by suitable coatings.

The pharmaceutical composition may comprise a therapeutically effective amount of a compound according to the invention.

The content of a compound of the invention in a pharmaceutical composition of the invention is e.g. from about 0.1 to about 100% w/w of the pharmaceutical composition.

The pharmaceutical compositions may be prepared by any of the method well known to a person skilled in pharmaceutical formulation.

In pharmaceutical compositions, the compounds are normally combined with a pharmaceutical excipient, i.e. a therapeutically inert substance or carrier.

The carrier may take a wide variety of forms depending on the desired dosage form and administration route.

The pharmaceutically acceptable excipients may be e.g. fillers, binders, disintegrants, diluents, glidants, solvents, emulsifying agents, suspending agents, stabilizers, enhancers, flavours, colors, pH adjusting agents, retarding agents, wetting agents, surface active agents, preservatives, antioxidants etc. Details can be found in pharmaceutical handbooks such as, e.g., Remington's Pharmaceutical Science or Pharmaceutical Excipient Handbook.

The following examples describe the preparation and activities of some specific agonists of the invention.

### Syntheses

### ABBREVIATIONS

| | |
|---|---|
| Fmoc: | fluorenyl-9-methoxycarbonyl |
| DMF | dimethylformamide |
| Pbf | 2,2,4,6,7-pentamethyldihydrobenzofuran-5-sulfonyl |
| Trt | Triphenylmethyl |
| Boc or tBoc | butoxycarbonyl |
| | |
| Dde | 1-(4,4-dimethyl-2,6-dioxocyclohexylidene)ethyl |
| HCTU | 1H-Benzotriazolium 1-[*bis*(dimethylamino)methylene] -5chloro-,hexafluorophosphate (1-),3-oxide |
| TFA | trifluoroacetic acid |
| MALDI | matrix-assisted laser-desorption ionization |
| NHS | *N*-hydroxy succinimide |

Peptidic agonists of the invention may be synthesized by solid phase peptide synthesis, using either an automated peptide synthesizer, or traditional bench synthesis. The solid support can be, for example, chlorotrityl (CI) or Wang (OH) resin, both of which are readily available commercially. The active groups of those resins react readily with the carboxyl group of an N-Fmoc amino acid, thereby covalently binding it to the polymer. The resin-bound amine may be deprotected by exposure to piperidine. A second N-protected amino acid may then be coupled to the resin-amino acid. These steps are repeated until the desired sequence is obtained. At the end of the synthesis, the resin-bound protected peptide may be deprotected and cleaved from the resin with trifluoroacetic acid (TFA). Examples of reagents facilitating the coupling new amino acids to the resin-bound amino acid chain are: tetra-methyluronium hexafluorophosphate (HATU), O-(1H-benzotriazole-1-yl)-N,N,N',N'-tetramethyluronium hexafluorophosphate (HBTU), O-(1H-benzotriazole-1-yl)-N,N,N',N'-tetramethyluronium tetrafluoroborate (TBTU), 1H-hydroxybenzotriazole (HOBt).

Peptide synthesis by solution chemistry rather than solid phase chemistry is also feasible.

Modification of a side-chain amino or carboxyl group of an amino acid in the peptide chain, for example to introduce a GAG-binding or other motif as described above, is a simple matter of selective protection and deprotection of other reactive side-chain groups not to be involved in the reaction.

The peptides referred to herein are made by solid phase synthesis, on PAL Peg-PS resin, (amide resin (amide resin Applied Bioscience, Warrington, UK GEN913401), using Fmoc chemistry with a 5x reagent excess. The coupling was performed by HCTU throughout, solvent DMF. Fmoc removal was performed with 20% piperidine in DMF, 10-15 minutes. However, these peptides could just as well have been synthesised by various other standard peptide synthesis methods such as tBOC chemistry and solution chemistry instead of solid state etc. The synthesis is illustrated by the following description, but peptides with which the invention is concerned were made by similar methods:

### Synthesis of [Cys2,Lys13,D-Cys27]PYY and analogs therof

In the following, the synthesis of the cyclic Y2 selective peptide [Cys2,Lys13,D-Cys27]PYY (SEQ ID No: 39), and the synthesis of analogs with either a GAG-binding motif, a serum protein binding motif or a polyethyleneglycol moiety attached at the epsilon amino group of Lys13, is described.

In general side group protection were standard Fmoc except for:

| | |
|---|---|
| Arg | = Fmoc Arg(Pbf) -OH |
| Asn, Gln | = Fmoc Asn(Trt)-OH |
| Thr, Ser, Asp, Glu, Tyr | = tButyl |
| Lys | = Fmoc Lys(tBoc)-OH |
| Ala-Ser 22-23 | = Fmoc AlaSer pseudoproline |

In the case of [Cys2,Lys13,D-Cys27]PYY the following special protection groups were used in order to obtain selective deprotection:

| | |
|---|---|
| Tyr 1 | = tBoc Tyr (tBu) -OH |
| Lys 13 | = Fmoc Lys(Dde)- OH |
| Cys 27 | = Fmoc DLys(Trt) -OH |

The peptide was synthesized by solid phase synthesis, on PAL Peg-PS resin (a resin which will generate the biologically important carboxyamide group upon cleavage), using Fmoc chemistry with a 5 fold molar reagent excess. The coupling was performed by HCTU throughout using DMF as solvent. Fmoc removal after each coupling step was performed with 20% piperidine in DMF for 10-15 minutes. The coupling was checked after each step by quantitative ninhydrin assay. In certain cases double couplings could be performed.

After the synthesis of the full length peptides, the protection group on the epsilon amino group of Lys13 was selectively removed by treatment with 2 % hydrazine in DMF for 15-20mins, while the peptide was still attached to the resin.

The resin was subsequently divided into different batches to generate the underivatized peptide as well as three different motif-modified peptides:
**1. [Cys2,Lys13,D-Cys27]PYY (SEQ ID No: 39) -** the "mother peptide" - was cleaved from the resin and deprotected by treatment with 95% trifluoroacetic acid : 2.5% water: 2.5% tripropyl silane for 2-3 hours.
   The intramolecular stabilizing disulfide bridge between Cys2 and D-Cys27 was generated by air oxidation by dissolving the peptide at <1mg/ml, in ammonium acetate pH 8.5 - 9 and stirring for 24-48 hours until no free thiols could be detected by the Ellman assay.
   The peptide was purified by reverse phase HPLC: Vydac 300A column, 250mm x 10mm column eluted with (Buffer A = 0.05% TFA in water; Buffer B = 60%MeCN: 40% water: 0.05% TFA) in a gradient of 30-80% buffer B over 20mins, 2ml/min. OD 215 nM was measured and the eluant containing the specific peptide was collected and lyophilized.
   The structure of the peptide was confirmed by mass spec, amino acid analysis and in a number of cases also by amino acid sequence analysis.
**2. [Cys2, N-(8-(8-gammaglutamoylamino-octanoylamino)-octanoyl)-Lys13,D-Cys27]PYY (SEQ ID No: 28)** -In order to attach a serum albumin binding motif, an 8-(8-gammaglutamoylamino-octanoylamino)-octanoyl group was linked to the free epsilon amino group of Lys13 after removal of the protecting Dde group while the peptide was still attached to the resin by peptide synthesis using protected aminooctanoic acid twice followed by protected gammaglutamic acid.
   The motif-modified peptide was cleaved from the resin, deprotected, cyclized and purified as described above for the "mother peptides".
**3. Fluorescein-[Cys2,N-{(Ala-Arg-Arg-Arg-Ala-Ala-Arg-Ala)₃}-Lys13,D-Cys27]PYY (SEQ ID No: 30) -** In order to attach a GAG-binding motif, the motif was build stepwise by peptide synthesis using the free epsilon amino group of Lys13, after removal of the Dde group, on the [Cys2,Lys13,D-Cys27]PYY peptide still attached to the resin, using standard Fmoc chemistry as described above in general: The GAG binding sequence attached in this way was Ala-Arg-Arg-Arg-Ala-Ala-Arg-Ala-Ala-Arg-Arg-Arg-Ala-Ala-Arg-Ala-Ala-Arg-Arg-Arg-Ala-Ala-Arg-Ala. A fluorescein tag group was added for in vitro and in vivo analytical purposes as its 5,6 isomer - NHS ester, 10x excess over 72 hours - to the N-terminus of the final GAG-binding sequence.
   The GAG binding-motif modified peptide was cleaved from the resin, deprotected, cyclized and purified as described above for the "mother peptide".
**4. [Cys2, N-{N'-(21-amino-4,7,10,13,16,19-hexaoxaheneicosanoyl)}-gammaglutamoyl-Lys13,D-Cys27]PYY (SEQ ID No: 37)**
   - In order to attach a polyethylenglycol moiety to the Y2 selective peptide, protected 21-amino-4,7,10,13,16,19-hexaoxaheneicosanoic-acid was joined by peptide synthesis using the free epsilon amino group of Lys13 after removal of the Dde grup on the [Cys2,Lys13,D-Cys27]PYY peptide still attached to the resin, followed by a protected gamma-glutamic acid.

The PEGylated peptide was cleaved from the resin, deprotected, cyclized and purified as described above for the "mother peptide".

Using the above methods, the agonists of the invention are synthesized

### Chemical analysis of peptides:

Analytical data for some of the peptides of the invention, synthesised by the above methods, are set out below, by way of illustration of the methods used and results achieved:
Data

| Peptide SEQ ID | Molecular formula | MW | Theoretical m/z | Measured Mass m/z | Rt min | Purity | HPLC Method |
|---|---|---|---|---|---|---|---|
| 13 | C186 H287 N55 056 S2 | 4253.8 | | 4252.4 | 15.5 | 98.3 | C |
| 36 | C190 H294 N56 056 S2 | 4298 | | 4297.0 | 12.5 | 90.0 | A |
| 15 | C97 H158 N32 )22 S2 | 2204.7 | | 2204.5 | 17.1 | 97.8 | C |

### Analytical HPLC method A

| | |
|---|---|
| Column | = Vydac C18 Peptide-Protein column, 250 x 4.6 mm |
| Buffer A | = 0.05% TFA in water |
| Buffer B | = 0.05% TFA in 100% MeCN |
| Gradient | = 0% B to 60% B in 20 min |
| Flow rate | = 1.00 mL/min |
| Wavelength | = 215 nm |

Mass spectroscopy = MALDI-TOF with gentisic acid or □cyanohydroxy cinnamic acid as matrix.

### Analytical HPLC method C

| | |
|---|---|
| Column | = Vydac C18 218TP54, 250 x 4.6 mm |
| Buffer A | = 20 mL of MeCN and 2 mL of TFA in Water (total volume 2000 mL) |
| Buffer B | = 2 mL of TFA in Water (total volume 2000 mL) |
| Gradient | = 25% B to 75% B over 27 min |
| Flow rate | = 1.00 mL/min |
| Wavelength | = 215 nm |
| Injection volume | = 10 µL |

### Biological Assays and Results

### I. IN VITRO ASSAYS TO DETERMINE PEPTIDE AFFINITY AND POTENCY

### Human Y2 receptor Affinity Assay

Affinity of test compounds for the human Y2 receptor is determined in a competition binding assay using 1251-PYY binding in COS-7 cells transiently transfected with the human Y2 receptor using a standard calcium phosphate transfection method.

Transfected COS-7 cells are transferred to culture plates one day after transfection at a density of 40 x 103 cells per well aiming at 5 - 8% binding of the radioactive ligand. Two days after transfection, competition binding experiments are performed for 3 hours at 4 C° using 25 pM of 1251-PYY (Amersham, Little Chalfont, UK). Binding assays are performed in 0.5 ml of a 50 mM Hepes buffer, pH 7.4, supplemented with 1 mM CaCl2, 5 mM MgCl2, and 0.1 % (w/v) bovine serum albumin and 100 µg/ml bacitracin. Non-specific binding is determined as the binding in the presence of 1 µM of unlabeled PYY. Cells are washed twice in 0.5 ml of ice-cold buffer and 0.5-1 ml of lysis buffer (8 M Urea, 2 % NP40 in 3 M acetic acid) is added and the bound radioactivity is counted in a gamma counter. Determinations are made in duplicate. Steady state binding is reached with the radioactive ligand under these conditions. EC50 values were calculated using a standard pharmacological data handling software, Prism 3.0 (graphPad Sofware, San Diego, USA).

### Human Y4 receptor Affinity Assay

Protocol as for the Y2 affinity assay, except that human Y4-transformed COS-7 cells are used, the competition assay uses 1251-PP, and PP is used for the determination of non-specific binding.

### Human Y1 receptor Affinity Assay

Protocol as for the Y2 affinity assay, except that human Y1-transformed COS-7 cells are used and are transferred to culture plates at a density of 1.5 x 106 cells per well. the competition assay uses 1251-NPY, and NPY is used for the determination of non-specific binding.

### Human Y5 receptor Affinity Assay

Protocol as for the Y2 affinity assay, except that human Y5-transformed COS-7 cells are used and are transferred to culture plates at a density of 5 x 105 cells per well. the competition assay uses 1251-NPY, and NPY is used for the determination of non-specific binding.

The results of testing NPY, PYY. PYY3-36, PP and some other agonists of the invention in the above affinity assays are given in Table 1:

**Table 1**

| Agonist | Y2 | | | Y1 | | | Y4 | | | Y5 | | |
|---|---|---|---|---|---|---|---|---|---|---|---|---|
| | IC50 nm | SEM | n | IC50 nm | SEM | n | IC50 nm | SEM | n | IC50 nm | SEM | n |
| NPY | 0.30 | 0.07 | 4 | 2.3 | 0.3 | 4 | 26.3 | 5.4 | 4 | 0.43 | 0.05 | 4 |
| PYY | 0.22 | 0.02 | 2 | 16 | 1 | 2 | 29.9 | 8.0 | 2 | 1.2 | 0 | 2 |
| PYY(3-36) | 0.20 | 0.03 | 10 | >1000 | | 1 2 | 343 | 42 | 4 | 22 | 5 | 2 |
| PP | >1000 | | 4 | >1000 | | 1 | 0.49 | 0.04 | 8 | 76 | | 1 |
| | | | | | | | | | | | | |
| SEQ ID NO: 13 | 0.79 | 0.18 | 3 | >1000 | | 2 | 657 | 29 | 3 | 116 | 50 | 2 |
| SEQ ID NO: 36 | 0.60 | | 1 | >1000 | | 1 | 300 | | 1 | 36 | | 1 |
| SEQ ID NO: 12 | 0.21 | 0.06 | 2 | >1000 | | 2 | 6.0 | | 1 | 0.87 | 0.13 | 2 |

### Human Y2 receptor Potency Assay

Potency of the test compounds on the human Y2 receptor is determined by performing dose-response experiments in COS-7 cells transiently transfected with the human Y2 receptor as well as a promiscuous G protein, Gqi5 which ensures that the Y2 receptor couples through a Gq pathway leading to an increase in inositol phosphate turnover.

*Phosphatidylinositol turnover -* One day after transfection COS-7 cells are incubated for 24 hours with 5 µCi of [3H]-myo-inositol (Amersham, PT6-271) in 1 ml medium supplemented with 10% fetal calf serum, 2 mM glutamine and 0.01 mg/ml gentamicin per well. Cells are washed twice in buffer, 20 mM HEPES, pH 7.4, supplemented with 140 mM NaCl, 5 mM KCI, 1 mM MgSO4, 1 mM CaCl2, 10 mM glucose, 0.05 % (w/v) bovine serum; and are incubated in 0.5 ml buffer supplemented with 10 mM LiCl at 37C for 30 min. After stimulation with various concentrations of peptide for 45 min at 37C, cells are extracted with 10 % ice-cold perchloric acid followed by incubation on ice for 30 min. The resulting supernatants are neutralized with KOH in HEPES buffer, and the generated [3H]-inositol phosphate are purified on Bio-Rad AG 1-X8 anion-exchange resin and counted in a beta counter. Determinations are made in duplicates. EC50 values were calculated using a standard pharmacological data handling software, Prism 3.0 (graphPad Sofware, San Diego, USA).

### Human Y4 receptor Potency Assay

Protocol as for the Y2 potency assay, except that human Y4-transformed COS-7 cells are used.

### Human Y1 receptor Potency Assay

Protocol as for the Y2 potency assay, except that human Y1-transformed COS-7 cells are used.

### Human Y5 receptor Potency Assay

Protocol as for the Y2 potency assay, except that human Y5-transformed COS-7 cells are used

The results of testing NPY, PYY, PYY3-36, PP and four of the agonists of the invention in the above potency assays are given in Table 2:

**Table 2**

| Agonist | Y2 | | | Y1 | | | Y4 | | | Y5 | | |
|---|---|---|---|---|---|---|---|---|---|---|---|---|
| | EC50 nm | SEM | n | EC50 nm | sem | n | EC50 nm | SEM | n | EC50 nm | sem | n |
| NPY | 1.5 | 1.5 | | 11 1.7 | 0.4 | 11 112 | | 10 | 6 | 8.9 | 2.2 | 11 |
| PYY | 0.23 | 0.06 | 8 | 0.6 | 1.11 | 5 | 36 | 6 | 5 | 8.5 | 1.1 | 5 |
| PYY(3-36) | 0.36 | 0.07 | 16 | 74 | 5 | 7 | >1000 | | 7 | 82 | 17 | 5 |
| PP | >1000 | | 8 | 83 | 13 | 5 | 0.64 | 0.07 | 17 | 30 | 7 | 5 |
| | | | | | | | | | | | | |
| SEQ ID NO: 13 | 0.48 | 0.16 | 5 | >1000 | | 1 | >1000 | | 5 | 198 | 44 | 4 |
| SEO ID NO: 36 | 1.9 | 1.6 | 2 | >1000 | | 2 | >1000 | | 2 | 888 | 505 | 2 |
| SEQ ID NO: 12 | 0.17 | 0.01 | 4 | 38 | 10 | 5 | 64 | 6 | 4 | 3.8 | 1.0 | 5 |

### II. IN VITRO ASSAYS TO DETERMINE PROTEIN STABILITY

An important measure for many of the peptides of the invention is the protein stability especially in respect of stability for example towards degradation by enzymes as the peptides have been designed to have increased stability as compared to for example PYY3-36 or even increased stability as compared to full length PYY and PP.

**Stability of the PP-fold -** is determined as the stability of the peptides towards degradation by endopeptidases which cleave for example in the loop region, which is relatively flexible as described *(*O'Hare, M. & Schwartz, T.W. 1990 In Degradation of Bioactive Substances: Physiology and Pathophysiology. J.Henriksen, ed. CRC Press, Boca Raton, Fl.). As a model enzyme, endoproteinase Asp-N (Pierce) is used. This enzyme cleave at the N-terminal side of Asp residues, for example between residues 9 and 10 (Asp) in PP. The peptides are incubated with an efficacious dose of endopeptidase Asp-N as indicated by the manufacturer in 0.01 M Tris/HCl buffer, pH 7.5 at room temperature and samples removed after various time periods over 24 hours. The samples are analysed by HPLC and the gradual degradation of the peptides is followed over time. The peptides are compared in respect of stability to PYY, PYY13-36 and PP.

**Stability towards aminopeptidases** - is determined as described above for endopeptidases but using aminopeptidase N and di-peptidylpeptidase IV in stead. Some of the peptides are specially designed to be resistant to these aminopeptidase, for example PYY2-36, the N-terminally acetylated peptide derivates, and peptide derivates alkylated with an albumin binding moiety at the N-terminus. The peptides are compared in respect of stability to PYY, PYY3-36 and PP.

### Ill. IN VITRO ASSAY TO DETERMINE BINDING TO GLYCOSAMINO GLYCANS (GAGS)

The ability of test compounds to bind to GAGs is monitored in an in vitro assay using immobilized heparin, i.e. for example either a HiTrap heparin-Sepharose column (Amersham Pharmacia Biotech, Uppsala, Sweden) or a heparin HPLC columns which are eluted with a 50-min linear gradient of 0-0.5 M NaCl in 50 mM sodium phosphate (pH 7.3) containing 2 mM DTT and 1 mM MgEDTA at a flow rate of 1 ml/min. For regeneration, the column was washed with 1 M NaCl in buffer A from 51-55 min. For initial analytical purposes a step-gradient of NaCl can be used.

### IV. IN VIVO STUDIES TO DETERMINE THE EFFECT OF THE PEPTIDES ON APPETITE, FOOD INTAKE AND BODY WEIGHT

### Effect of Y2 over Y1 and Y4 selective agonists on acute food intake in mice

The ddy strain of mice were used, 34-37 g and 8-9 weeks of age (Japan SLC, Shizuuoka, Japan). The mice were individually house in a regulated environment (22 C, 55 % humidity) in a 12-hour light-dark cycle with light on at 7 AM. Food and water were available ad libitum except just before experiments (see below). The mice were acclimatized to subcutaneous injections during the week before experiments started. Mice were used once each in the experiments. Just before administration the peptides were diluted in physiological saline and administered in 100 µL volume for subcutaneous administration. Results are expressed as mean +/- SE. Analysis of variance followed by Bonferroni's test were used to assess differences among groups.

Mice were deprived of food but with free access to water for 16 hours prior to the actual test and experiments were started at 10 AM on the following day. A standard diet was used (CLEA Japan, Inc., Tokyo, Japan) and food intake was measured by subtracting uneaten food from the initially premeasured food after administration and checking for food spillage. Eight animals in each group receiving either saline, 3 µg PYY3-36, 30 µg PYY3-36, 10 µg test compound, or 100 µg test compound.

The results for [Cys2,D-Cys27]-PYY (Seq ID No: 13) as test compound are shown in Fig. 2.

### SEQUENCES:

NPY (SEQ ID No: 1)
H2N-Tyr--Pro-Ser-Lys-Pro-Asp-Asn-Pro-Gly-Glu-Asp-Ala-Pro-Ala-Glu-Asp-Met-Ala-
Arg-Tyr-Tyr-Ser-Ala-Leu-Arg-His-Tyr-Ile-Asn-Leu-Ile-Thr-Arg-Gln-Arg-Tyr-CONH2

PYY (SEQ ID No: 2)
H2NTyr-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Leu-Val-Thr-Arg-Gln-Arg-Tyr-CONH2

PP (SEQ ID NO: 3)
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Met-Leu-Thr-Arg-Pro-Arg-Tyr-CONH2

[Lys4,Gln34]PP (SEQ ID No: 12)
H2N-Ala-Pro-Leu-Lys-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Met-Leu-Thr-Arg-Gln-Arg-Tyr-CONH2

[Cys2,D-Cys27]PYY (SEQ ID No: 13)
S
|
H2N-Tyr-Cys-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-(D-Cys)-Leu-Asn-Leu-Val-Thr-Arg-Gln-Arg-Tyr-CONH2
|
S

[Cys2,D-Cys27]NPY (SEQ ID No: 14)
S
H2N-Tyr-Cys-Ser-Lys-Pro-Asp-Asn-Pro-Gly-Glu-Asp-Ala-Pro-Ala-Glu-Asp-Met-Ala-
Arg-Tyr-Tyr-Ser-Ala-Leu-Arg-His-(D-Cys)-Ile-Asn-Leu-Ile-Thr-Arg-Gln-Arg-Tyr-
CONH2
S

[Cys2,Ile3,D-Cys27,Val31]NPY (SEQ ID No: 15)
S
H2N-Tyr-Cys-Ile-Lys-Pro-Asp-Asn-Pro-Gly-Glu-Asp-Ala-Pro-Ala-Glu-Asp-Met-Ala-
Arg-Tyr-Tyr-Ser-Ala-Leu-Arg-His-(D-Cys)-Ile-Asn-Leu-Val-Thr-Arg-Gln-Arg-Tyr-
CONH2
S

[Lys4,Leu17,Gln34]PP (SEQ ID No: 23)
H2N-Ala-Pro-Leu-Lys-Pro-Val-Tyr-Pro-Gly-Asp-Asn-Ala-Thr-Pro-Glu-Gln-Leu-Ala-
Gln-Tyr-Ala-Ala-Asp-Leu-Arg-Arg-Tyr-Ile-Asn-Met-Leu-Thr-Arg-Gln-Arg-Tyr-CONH2

[Lys4,Leu17,Leu30,Gln34]PP (SEQ ID No: 24)
H2N-Ala-Pro-Leu-Lys-Pro-Val-Tyr-Pro-Gly-Asp-Asn-Ala-Thr-Pro-Glu-Gln-Leu-Ala-
Gln-Tyr-Ala-Ala-Asp-Leu-Arg-Arg-Tyr-Ile-Asn-Leu-Leu-Thr-Arg-Gln-Arg-Tyr-CONH2

[Lys4,Nle17,Nle30,Gln34]PP (SEQ ID No: 25)
H2N-Ala-Pro-Leu-Lys-Pro-Val-Tyr-Pro-Gly-Asp-Asn-Ala-Thr-Pro-Glu-Gln-Nle-Ala-Gln-
Tyr-Ala-Ala-Asp-Leu-Arg-Arg-Tyr-Ile-Asn-Nle-Leu-Thr-Arg-Gln-Arg-Tyr-CONH2

N-acetyl[Cys2-DCys27]PYY2-36 (SEQ ID No: 27)
S
CH3CONH-Cys-Ile-Lys-Pro-Glu-Ala-Pro-Gly-Glu-Asp-Ala-Ser-Pro-Glu-Leu-Asn-Ala-
Arg-Tyr-Tyr-Ala-Ser-Leu-Arg-His-(D-Cys)-Leu-Asn-Leu-Val-Thr-Arg-Gln-Arg-Tyr-
CONH2
S

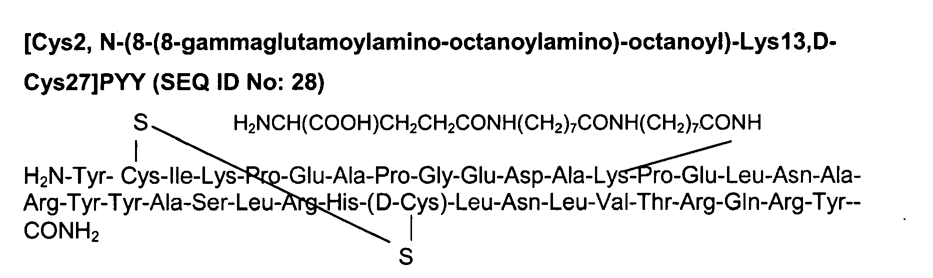
[Cys2, N-(8-(8-gammaglutamoylamino-octanoylamino)-octanoyl)-Lys13,D-
Cys27]PYY (SEQ ID No: 28)
S          H2NCH(COOH)CH2CH2CONH(CH2)7CONH(CH2)7CONH
|
H2N-Tyr- Cys-Ile-Lys-Pro-Glu-Ala-Pro-Gly-Glu-Asp-Ala-Lys-Pro-Glu-Leu-Asn-Ala-
Arg-Tyr-Tyr-Ala-Ser-Leu-Arg-His-(D-Cys)-Leu-Asn-Leu-Val-Thr-Arg-Gln-Arg-Tyr--
CONH2
|
S

[Cys2,N-{(Ala-Arg-Arg-Arg-Ala-Ala-Arg-Ala)3}-Lys13,D-Cys27]PYY (SEQ ID No:
30)
S
Ala-Arg-Arg-Arg-Ala-Ala-Arg-Ala)3CONH
H2N-Tyr- Cys-Ile-Lys-Pro-Glu-Ala-Pro-Gly-Glu-Asp-Ala-Lys-Pro-Glu-Leu-Asn-Ala-
Arg-Tyr-Tyr-Ala-Ser-Leu-Arg-His-(D-Cys)-Leu-Asn-Leu-Val-Thr-Arg-Gln-Arg-Tyr--
CONH2
S

[Cys2,Ile3,D-Cys27,Leu28,Val31]NPY (SEQ ID No: 32)
S
H2N-Tyr-Cys-Ile-Lys-Pro-Asp-Asn-Pro-Gly-Glu-Asp-Ala-Pro-Ala-Glu-Asp-Met-Ala-
Arg-Tyr-Tyr-Ser-Ala-Leu-Arg-His-(D-Cys)-Leu-Asn-Leu-Val-Thr-Arg-Gln-Arg-Tyr-
CONH2
S

[Cys2,Ile3,Nle17,D-Cys27,Nle28,Val31]NPY (SEQ ID No: 33)
S
S
H2N-Tyr-Cys-Ile-Lys-Pro-Asp-Asn-Pro-Gly-Glu-Asp-Ala-Pro-Ala-Glu-Asp-Nle-Ala-
Arg-Tyr-Tyr-Ser-Ala-Leu-Arg-His-(D-Cys)-Nle-Asn-Leu-Val-Thr-Arg-Gln-Arg-Tyr-
CONH2
S

N-(N'-tetradecanoyl)-gammaglutamoyl-[Cys2, D-Cys27]PYY (SEQ ID No: 35)
S
CH3(CH2)12CONHCH(COOH)CH2CH2CONH -Tyr-Cys-Ile-Lys-Pro-Glu-Ala-Pro-Gly-
Glu-Asp-Ala-Ser-Pro-Glu-Glu-Leu-Asn-Arg-Tyr-Tyr-Ala-Ser-Leu-Arg-His-(D-Cys)-
Leu-Asn-Leu-Val-Thr-Arg-Gln-Arg-Tyr-CONH2
S

[Cys2, D-Cys27]PYY2-36 (SEQ ID No: 36)
S
H2N-Tyr-Cys-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-(D-Cys)-Leu-Asn-Leu-Val-Thr-Arg-Gln-Arg-Tyr-CONH2
S

[Cys2, N-{N'-(21-amino-4,7,10,13,16,19-hexaoxaheneicosanoyl)}-
gammaglutamoyl-Lys13,D-Cys27]PYY (SEQ ID No: 37)

S
H2N(CH2CH2O)6CH2CH2CONHCH(COOH)CH2CH2CONH
H2N-Tyr-Cys-Ile-Lys-Pro-Glu-Ala-Pro-Gly-Glu-Asp-Ala-Lys-Pro-Glu-Glu-Leu-Asn-Arg-
Tyr-Tyr-Ala-Ser-Leu-Arg-His-(D-Cys)-Leu-Asn-Leu-Val-Thr-Arg-Gln-Arg-Tyr-CONH2
S

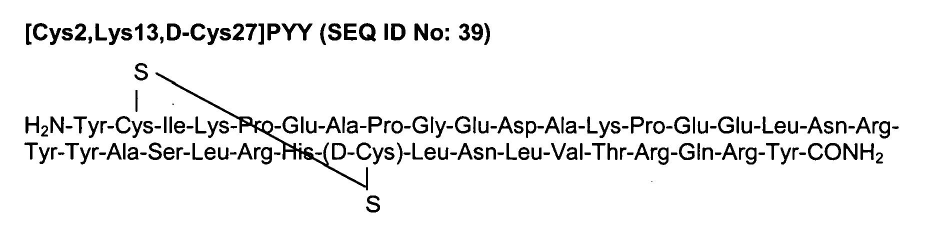
[Cys2,Lys13,D-Cys27]PYY (SEQ ID No: 39)

S
H2N-Tyr-Cys-Ile-Lys-Pro-Glu-Ala-Pro-Gly-Glu-Asp-Ala-Lys-Pro-Glu-Glu-Leu-Asn-Arg-
Tyr-Tyr-Ala-Ser-Leu-Arg-His-(D-Cys)-Leu-Asn-Leu-Val-Thr-Arg-Gln-Arg-Tyr-CONH2
S

## Claims

1. A Y receptor agonist selective for the Y2 receptor over the Y1 and Y4 receptors, selected from the group consisting of:
[Lys4,Leu17,Gln34]PP (SEQ ID No: 23)
[[Cys2, D-Cys27]PYY2-36 (SEQ ID No: 36)
Lys4,Leu17,Leu30,Gln34]PP (SEQ ID No: 24)
[Lys4,Nle17,Nle30,Gln34]PP (SEQ ID No: 25)
[Lys4,Gln34]PP (SEQ ID No: 12)
[Cys2,D-Cys27]PYY (SEQ ID No: 13)
[Cys2,D-Cys27]NPY (SEQ ID No: 14)
[Cys2,Ile3,D-Cys27,Val31]NPY (SEQ ID No: 15)
[Cys2,Ile3,D-Cys27,Leu28,Val31]NPY (SEQ ID No: 32)
[Cys2,Ile3,Nle17,D-Cys27,Nle28,Val31]NPY (SEQ ID No: 33)
and N-acylated and conservatively substituted analogues thereof.

2. A Y receptor agonist as claimed in claim 1 which is acetylated at its N-terminus to confer resistance to aminopeptidase activity.

3. A Y receptor agonist as claimed in claim 1 or claim 2 which comprises a serum albumin binding motif, or a glycosaminoglycan (GAG) binding motif, or a helix inducing motif, or which is PEGylated.

4. A pharmaceutical composition comprising one or more Y receptor agonists as defined in any of claims 1 to 3 together with a pharmaceutically acceptable excipient.

5. The use of a Y receptor agonist as claimed in any of claims 1 to 3 for the regulation of energy intake or energy metabolism, for the induction of angiogenesis, or for the treatment of bulimia, bulimia nervosa, Syndrome X (metabolic syndrome), diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, coronary artery disease, myocardial infarction, peripheral vascular disease, stroke, thromboembolic disease, hypercholesterolemia, hyperlipidemia, gallbladder disease, osteoarthritis, sleep apnea, reproductive disorders such as polycystic ovarian syndrome, or cancer of the breast, prostate, or colon.
